# EUROPÄISCHE PATENTSCHRIFT

(11) **EP 1 427 817 B1**
(45) Veröffentlichungstag und Bekanntmachung des Hinweises auf die Patenterteilung: **05.05.2010**
(21) Anmeldenummer: 02777068.4
(22) Anmeldetag: 11.09.2002
(51) Int. Cl.: C12N 7/00, A61K 39/12

(54) **VERMEHRUNG VON VIREN IN ZELLKULTUR**
MULTIPLICATION OF VIRUSES IN A CELL CULTURE
MULTIPLICATION DE VIRUS DANS UNE CULTURE CELLULAIRE

(30) Priorität: 12.09.2001 DE 10144903
(43) Veröffentlichungstag der Anmeldung: 16.06.2004
(73) Patentinhaber: Novartis Vaccines and Diagnostics GmbH, 35041 Marburg (DE)
(72) Erfinder: VORLOP, Jürgen, 35041 Marburg (DE); FRECH, Christian, 68219 Mannheim (DE); LÜBBEN, Holger, 35083 Wetter (DE); GREGERSEN, Jens-Peter, 35083 Wetter (DE)
(74) Vertreter: UEXKÜLL & STOLBERG
(86) Internationale Anmeldenummer: PCT/EP2002/010207
(87) Internationale Veröffentlichungsnummer: WO 2003/023025

(56) Entgegenhaltungen:
- WO-A-97/37000
- WO-A2-01/02548
- TREE J A ET AL: "Comparison of large-scale mammalian cell culture systems with egg culture for the production of influenza virus A vaccine strains" VACCINE, BUTTERWORTH SCIENTIFIC. GUILDFORD, GB, Bd. 19, Nr. 25-26, 14. Mai 2001 (2001-05-14), Seiten 3444-3450, XP004238940 ISSN: 0264-410X
- MERTEN O W ET AL: "Production of influenza virus in serum-free mammalian cell cultures." DEVELOPMENTS IN BIOLOGICAL STANDARDIZATION. SWITZERLAND 1999, Bd. 98, 1999, Seiten 23-37;discussion 73 - 74, XP001097587 ISSN: 0301-5149
- KESSLER N ET AL: "Suitability of MDCK cells grown in a serum-free medium for influenza virus production." DEVELOPMENTS IN BIOLOGICAL STANDARDIZATION. SWITZERLAND 1999, Bd. 98, 1999, Seiten 13-21;discussion 73 - 74, XP001097586 ISSN: 0301-5149
- MERTEN O-W ET AL: "PRODUCTION OF INFLUENZA VIRUS IN CELL CULTURES FOR VACCINE PREPARATION" ADVANCES IN EXPERIMENTAL MEDICINE AND BIOLOGY, SPRING ST., NY, US, Bd. 397, 1996, Seiten 141-151, XP002039317 ISSN: 0065-2598
- PERRIN P ET AL: "An experimental rabies vaccine produced with a new BHK-21 suspension cell culture process: use of serum-free medium and perfusion-reactor system" VACCINE, BUTTERWORTH SCIENTIFIC. GUILDFORD, GB, Bd. 13, Nr. 13, 1995, Seiten 1244-1250, XP004057477 ISSN: 0264-410X
- SAWICKI S.G. ET AL: 'Persistent infection of cultured cells with mouse hepatitis virus (MHV) results from the epigenetic expression of the MHV receptor' JOURNAL OF VIROLOGY Bd. 69, Nr. 9, September 1995, Seiten 5535 - 5543
- PARDOE I.U. ET AL: 'Persistent infection of K562 cells by Encephalomyocarditis Virus' JOURNAL OF VIROLOGY Bd. 64, Nr. 12, Dezember 1990, Seiten 6040 - 6044
- LLOYD R.E.; BOVEE M.: 'Persistent infection of human erythroblastoid cells by poliovirus' VIROLOGY Bd. 194, 1993, Seiten 200 - 209

## Beschreibung

Die Erfindung betrifft Verfahren zur Vermehrung von ausgewählten Viren in Zellkultur, bei denen man MDCK- Zellen mit einem Virus infiziert und nach der Infektion die Zellen in Zellkultur unter Bedingungen zieht, die eine Vermehrung der Viren und gleichzeitig eine gezielte weitere, mindestens 2-fache Vermehrung der Zellen ermöglichen. Die Erfindung betrifft ferner die Verwendung der so erhältlichen Viren oder der von diesen exprimierten Proteine für die Herstellung von Arznei- und Diagnosemitteln.

Infektionskrankheiten, insbesondere virale Infektionen, haben nach wie vor große medizinische Bedeutung. Es besteht daher unverändert die Notwendigkeit bessere Verfahren zur Verfügung zu stellen, mittels derer Viren in Kultur vermehrt werden können, um die Erforschung der Viren und die Herstellung von Impfstoffen zu ermöglichen. Insbesondere die Produktion von Impfstoffen gegen virale Infektion erfordert üblicherweise die Vermehrung und Isolierung großer Mengen des betreffenden Virus.

In Abhängigkeit des jeweiligen Virus werden im Stand der Technik unterschiedliche Wirtssysteme und Kultivierungsbedingungen zur Virusvermehrung verwendet. Als Wirtssysteme kommen standardmäßig Wirtstiere, embryonierte Hühnereier primäre Gewebezellkulturen oder etablierte, permanente Zelllinien zur Anwendung (Rolle und Mayr [Hersg.], Mikrobiologie, Infektions- und Seuchenlehre, 1978; Mahy [Hersg.], Virology, A Practical Approach, 1985; Horzinek [Hersg.] Kompendium der allgemeinine Virologie, 1985).

Die Virusvermehrung in embryonierten Hühnereiern ist mit hohem Zeit- und Kostenaufwand verbunden. Die Eier müssen vor der Infektion bebrütet und anschließend auf Lebensfähigkeit der Embryonen hin getestet werden. Nur lebende Embryonen sind in der Lage sind, Viren zu vermehren. Nach erfolgter Infektion mit dem zu vermehrenden Virus und weiterer Bebrütung werden die Embryonen schließlich abgetötet. Die aus den Eiern isolierbaren Viren werden von Verunreinigungen befreit und konzentriert. Da die Vermehrung von Viren in bebrüteten Eiern nicht unter strikt sterilen Bedingungen möglich ist, müssen kontaminierende pathogene Mikroorganismen aus den Isolaten entfernt werden, sofern diese einer medizinischen oder diagnostischen Applikation zugänglich sein sollen.

Eine Alternative zur Vermehrung von Viren in Hühnereiern bieten eukaryontische Wirtszellen definierter Zelllinien (Gregersen, J.P., Pharmazeutische Biotechnologie, Kayser und Müller [Hersg.], 2000, Seiten 257-281). Zahlreiche Zelllinien sind jedoch aufgrund von persistenten Fremdvirus-Kontaminationen bzw. aufgrund mangelnder Beweise für die Virusfreiheit, unklarer Herkunft und Historie nicht für die Herstellung von Impfstoffen oder ähnlichen medizinisch verwendbaren Präparaten geeignet.

Die im Stand der Technik verwendeten Verfahren zur Vermehrung von Viren in Zellkultur weisen alle dasselbe Grundschema auf, bei dem man zunächst die Zellen in Abwesenheit des Virus vermehrt, anschließend das Virus zugibt und unter Bedingungen vermehrt, bei denen keine signifikante Vermehrung der Zellen erfolgt, und die Kultur nach maximaler Vermehrung der Viren erntet.

Beispielsweise wurden die aus Nierenzellen von Affen abgeleiteten Vero-Zellen zur Vermehrung einzelner Viren (Poliovirus, Tollwutvirus) zur Impfstoffherstellung eingesetzt wird. Diese Zellen sind bei diversen Zellbanken (wie beispielsweise der American Type Culture Collection, ATCC) erhältlich und werden darüber hinaus auch von der Weltgesundheitsorganisation (WHO) aus einer geprüften Zellbank für die medizinische Forschung zur Verfügung gestellt.

Bei diesen Vero-Zellen handelt es sich um adhärente Linien, die für ihr Wachstum Trägerflächen, wie beispielsweise Glasflaschen, Plastikkulturschalen oder Plastikflaschen benötigen. Bei einer Kultur entsprechender Zellen im Fermenter erfolgt das Wachstum an sogenannten Microcarriern, d.h. in der Regel kleinen Kunststoffkügelchen, auf deren Oberfläche die Zellen wachsen können.

Bekannt ist, dass neben den obengenannten Vero-Zellen beispielsweise auch adhärente BHK- ("Baby Hamster Kidney") und adhärente MDCK- ("Mandine Darby Canine Kidney") Zellen und andere Zellen mehrere Virusarten aktiv vermehren können und für den Einsatz als Substrat zur Herstellung pharmazeutischer Produkte eingesetzt werden oder deren Einsatz erwogen wird. In der MDCK-Zelllinie ATCC CRL34 (NBL-2) wurden neben Influenzaviren experimentell auch das Vesicular Stomatitis Virus, das Coxsakkievirus B5 (nicht aber B3 oder B4), das Rheovirus-Typ 2 und 3, das Adenovirus-Typ 4 und 5 sowie Vaccinia-Viren vermehrt. Alle entsprechenden Veröffentlichungen richten sich jedoch ausschließlich auf adhärente Kulturen (vgl. Produktinformation ATCC). Für die Vermehrung von größeren Zellmengen ist jedoch die Suspensionskultur bevorzugt, wobei nur die lymphoiden und manche transformierten Zellen in diesem System bisher vermehrt werden können (Lindl [Hersg.], Zell- und Gewebekultur, 2000, Seiten 173 ff.). Eine MDCK-Zelllinie, die in Protein-freien Kulturmedien in Suspension zu wachsen vermag, wird in WO 97/37000 offenbart. Die Vermehrung von Influenzaviren unter Verwendung der entsprechenden Wirtzellen wird ebenfalls beschrieben.

Neben der Auswahl eines geeigneten Zell- bzw. Wirtssystems sind die Kultivierungsbedingungen, unter denen ein Virusstamm vermehrt wird für das Erreichen einer akzeptablen hohen Ausbeute ebenfalls von großer Bedeutung. Um die Ausbeute des erwünschten Virusstammes zu maximieren, müssen daher sowohl Wirtssystem als auch Kultivierungsbedingungen spezifisch angepaßt werden, um für den gewünschten Virusstamm günstige Umgebungsbedingungen zu erreichen. Um eine hohe Ausbeute der uhteschiedliche Virusstämme zu erreichen, wird daher ein System benötigt, das optimale Wachstumsbedingungen schafft. Viele Viren sind auf spezielle Wirtssysteme beschränkt, von den einige hinsichtlich der Virusausbeute sehr ineffizient sind. Effiziente Produktionssysteme basieren zudem oft auf Adaptionen der Viruspopulation an das jeweilige Kultursystem, oft unter Verwendung von Zwischenstufen mit Verwendung anderer Wirtssysteme und unter Anwendung von Proteinzusätzen - meist Serum - tierischen oder menschlichen Ursprungs.

Einschlägig erfahrenen Personen ist ferner bekannt, dass fast alle Zellkulturen nach anfänglicher Vermehrung unter Zusatz von Serum oder anderen Wachstumsfaktoren zumindest für eine gewisse Zeit ohne Serum- oder Proteinzusätze gehalten werden können. So kann beispielsweise eine beliebige Zellkultur zum Zeitpunkt der Virusinfektion oder kurz vor der Ernte auf Medium ohne Serum oder Proteinzusätze umgestellt werden und bis zur Ernte weiter gehalten werden. Dies ist seit Jahren gängige Praxis, um unter Vermeidung bzw. Reduktion von Fremdproteinen Virusmaterial für Impfstoffe oder diagnostische Tests zu gewinnen. Impfstoffe aus Zellkulturen, die ohne diese Praxis während der Infektionsphase unter Serumzusatz gehalten wurden, werden größere Probleme haben, zur Anwendung bei Mensch oder Tier zugelassen zu werden, da sich die Serumbestandteile kaum noch hinreichend entfernen lassen (vgl. WHO-Empfehlungen "Proposed requirements for Measles Vaccine" (Live), Requirements for Biological Subtances No.12, Revised 1978) .

Bekannt ist auch, dass manche Viren sich in proteinhaltigen Medien nur sehr schlecht oder gar nicht vermehren lassen. Es handelt sich dabei um solche Viren, die für ihre Vermehrung in Kultursystemen auf die Aktivität proteinspaltende Enzyme (Proteasen) angewiesen sind. Da diese Proteasen durch die Proteinzusätze zum Medien kompetitiv gehemmt werden, verbietet sich hier logischerweise der Zusatz von Proteinen zumindest ab dem Zeitpunkt der Infektion oder Produktionsphase. Beispiele für Viren, die üblicherweise unter Zusatz von Proteasen und somit zur Erzielung guter Ausbeuten möglichst ohne Proteinzusätze zum Infektionsmedium vermehrt werden müssen, sind Influenzaviren und Rotaviren. Andere Virusarten wie z.B. Paramyxoviren und Reoviren können ebenfalls bei der Vermehrung von möglichst proteinarmen Medien profitieren (Ward et al. (1984) J.Clin.Microbiol. 748-753 "Efficiency of Human Rotavirus Propagation in Cell Culture"). WO 96/15231 schlägt vor, Vero- und andere Zellen in Zellkultur zu ziehen, wobei ein Medium verwendet werden soll, dass ohne die üblichen Protein-Zusätze auskommt.

Andere Viren lassen sich unabhängig von der Mediumzusammensetzung und den Kulturbedingungen notorisch schlecht vermehren, beispielsweise Tollwut-, Rota-, Pneumo- oder Hepatitis A Viren (Provost und Hillemann, Proc. Soc. Exp. Bio. Med., 160:213-221 (1979); und Rolle und Mayr a.a.O.).

Nach der Vermehrung des Virus wird dieser üblicherweise aus der Kultur isoliert. Es sind im Stand der Technik zahlreiche Verfahren bekannt, mittels derer Viren, virale Expressionsprodukte oder andere Proteine nach der Vermehrung aus dem Medium und/oder den Zellen isoliert werden können (Gregersen a.a.O; Mahy a.a.O.; Reimer C., et al., Journal of Virology, Dec. 1967, p. 1207-1216; Navarro del Canizo, A., et al., Applied Biochemistry and Biotechnology, Vol. 61, 1996, 399; Prior, C., et al., BioPharm, Oct. 1996, 22; Janson, Jan-C. and Rydén, L. [Hersg.], Protein Purification, 1997; und Deutscher M. [Hersg.], Methods in Enzymology, Vol. 182, 1990).

Aufgrund des schematischen Ablaufs der Verfahren (Zellvermehrung, Virusvermehrung, Ernte) ermöglichen die im Stand der Technik bekannten Verfahren jedoch lediglich eine begrenzte Virusvermehrung und Ernte.

WO 01/02548 beschreibt ein Verfahren zur Vermehrung lytische Organismen in einem Hohlfaser-Bioreaktor.

Die der vorliegenden Erfindung zugrunde liegende Aufgabe besteht somit darin, Verfahren zur Vermehrung von Viren in Zellkultur zur Verfügung zu stellen; die eine größere Virusvermehrung und vereinfachte Ernte größerer Mengen an Virus ermöglichen.

Diese Aufgabe wurde nunmehr durch Verfahren zur Herstellung eines Arznei- oder Diagnosemittels gelöst, Schritte umfassend, bei denen man
(a) MDCK-Zellen mit einem Virus infiziert;
(b) nach der Infektion die MDCK-Zellen in Zellkultur unter Bedingungen zieht, die eine Vermehrung der Viren und gleichzeitig eine gezielte weitere, mindestens 2-fache Vermehrung der Zellen durch Vergröβerung des gesamten Kulturvolumens ermöglichen,
wobei das Virus ausgewählt ist aus der Gruppe bestehend aus Viren der europäischen Frühsommer-Meningoenzephalitits (FSME) sowie der verwandten östlichen (russischen oder anderen) Formen, Japanisches Enzephalitis-Virus, Dengue Virus, Gelbfiebervirus, Tollwutviren, Hepatitis A Viren, oder Rotaviren.

Überraschenderweise wird ein wesentlich verbessertes Verfahren zur Vermehrung von Viren in Zellkultur erhalten, wenn man den Ablauf des Verfahrens grundlegend dahingehend verändert, das ein Wachstum der Zellen auch während der Vermehrung des Virus ermöglicht wird. Dies hat den Vorteil, dass in kürzerer Zeit signifikant mehr Virus erhalten werden kann. Ferner wird die Auslastung der Anlagen für die Zellkultur verbessert.

Erfindungsgemäß wird die Zellkultur so durchgeführt, dass sich die Zellen nach der Infektion um mindestens das 2-fache oder 5-fache, vorzugsweise mindestens das 10-fache erhöht.

Die Vermehrung der Zellen bewirkt auch, dass die Kultur über einen Zeitraum von mindestens 7 Tagen nach der Infektion durchgeführt werden kann, vorzugsweise werden die Zellen und viren jedoch über mindestens 21, 28 oder 35 Tage in Zellkultur vermehrt.

Je nach der Verfahrensführung kann es vorteilhaft sein, während der Vermehrung der Viren und der Zellen mindestens einmal frisches Medium, Medium-Konzentrat oder Medienbestandteile zuzugeben oder mindestens einen Teil der Viren und Zellen in ein Kulturgefäß zu überführen, welches frisches Medium, Medium-Konzentrat oder Medienbestandteile enthält. Es ist jedoch bevorzugt, während der Vermehrung der Viren und der Zellen mindestens einmal frisches Medium, Medium-Konzentrat oder Medienbestandteile zuzugeben.

Die Zugabe des Mediums, des Medium-Konzentrates oder der Medienbestandteile wird vorzugsweise mindestens einmal oder mehrfach wiederholt.

Gemäß einer weiteren bevorzugten Ausführungsform der vorliegenden Erfindung wird während der Kultur der infizierten Zellen das Kulturmedium gegen frisches Kulturmedium ausgetauscht oder das Kulturvolumen durch Zugabe von frischem Kulturmedium vergrößert. Der Austausch bzw. die Ergänzung des Kulturmediums kann auch durch Mediumkonzentrat oder Mediumbestandteile wie beispielsweise Aminosäuren, Vitamine, Lipid-Fraktion, Phosphate, o.ä. Substanzen erfolgen. Diese Schritte können während der Kultur der Zellen auch mehrfach durchgeführt werden.

Dies ermöglicht die Steigerung der Virusausbeute durch mehrfache Virusernten aus Kulturüberständen, sowie besonders durch Vergrößerung des gesamten. Kulturvolumens und dabei auch der Zellzahl durch Zusatz frischen Mediums. Entsprechende Mehrfachernten stellen einen signifikanten Vorteil der erfindungsgemäßen Verfahren dar, da die Ausbeute des Systems wesentlich verbessert wird.

In den Verfahren der vorliegenden Erfindung werden MDCK-Zellen für die Vermehrung der Viren verwendet.

Bei den Zellen, die in den erfindungsgemäßen Verfahren zur Anwendung gelangen, handelt es sich um Zellen, welche die Eigenschaft haben, in Suspensionskultur oder als adhärente Zellen zu wachsen. Als Zellen, die in Suspensionskultur wachen werden Linien bezeichnet, die auch in Abwesenheit von Trägerteilchen im Fermenter im technischen Maßstab wachsen können, was gegenüber anderen Zellen erhebliche Vorteile bei der Handhabung der Kulturen, beim Vergrößern des Maßstabs der Kulturen und bei der Vermehrung von Viren aufweist. Verfahren zur Adaptation von MDCK-Zellen an die Suspensionskultur sind im Stand der Technik bekannt (WO 97/37000). Die MDCK-Zellen können beispielsweise von der Zelllinie MDCK-33016 abstammen.

Gemäß einer weiteren Ausführungsform der Erfindung werden Zellen verwendet, die sowohl vor als auch nach der Infektion die Eigenschaft aufweisen, adhärent und als Suspensionskultur zu wachsen. Diese Ausführungsform weist den besonderen Vorteil auf, dass ein Zellkultursystem und somit auch ein Medium für die Entwicklung der Zellen vom Labor-Maßstab bis zur großtechnischen Produktion verwendet werden kann. Entsprechende Systeme vereinfachen die Arzneimittelzulassung erheblich, da nur die Sicherheit eines einzelnen Zellkultursystems geprüft werden muß.

Das Virus kann ein Genom aus doppelsträngiger Ribonukleinsäure (dsRNS) oder einzelsträngiger Ribonukleinsäure aufweisen. Die einzelsträngigen Ribonukleinsäure-Moleküle können dabei die Polarität einer Boten-RNS ("messenger-RNS) aufweisen, RNS(+), oder entgegengesetzter Polarität sein, RNS(-).

Bei dem Virus kann es sich um ein unten genanntes Virus handeln. Die Viren, die im Rahmen des erfindungsgemäßen Verfahrens Anwendung finden, können über verschiedenen Sammlungen, wie z.B der ATCC (American Type Culture Collection) oder der ECACC (European Collection of Animal Cell Cultures) bezogen werden. In der Regel wird dabei auf existierende Produktionsstämme oder bereits in Zellkultur vorvermehrte Virusstämme zurückgegriffen. Es können ferner eigene Isolate etabliert werden, sofern diese sich für die jeweilige Applikation besser eignen. Gemäß einer Ausführungsform ist das Virus, welches in das Verfahren eingesetzt wird, ausgewählt aus der Gruppe bestehend aus: Hepatitis A-Virus, Japanisches Enzephalitis-Virus, Viren der europäischen Frühsommer-Meningoenzephalitits sowie der verwandten östlichen (russischen oder anderen) Formen, Dengue Virus, Gelbfiebervirus,

Gemäß einer weiteren Ausführungsform der vorliegenden Erfindung kann das Genom des Virus eine Nukleinsäuresequenz umfassen, die für ein heterologes, funktionales Protein mit einer Größe von mindestens 10 kDa kodiert. Im Stand der Technik sind zahlreiche Vektoren für die Expression von heterologen Proteinen bekannt, die auf einem viralen Genom, beispielsweise auf einem Herpes-, Vaccinia- oder Adenovirus-Genom, basieren (Galler R. et al., Braz.J.Med.Biol. Res., 1997 Feb., 30(2): 157-68; Willemse MJ. et al., Vaccine 1996 Nov., 14(16): 1511-6; Efstathiou S.; Minson AC., Br.Med.Bull., 1995 Jan. 51(1): 45-55; Hammerschmidt W., Curr.Opin.Mol.Ther., 2000 Oct., 2(5):532-9; Graham Fl.; Prevec, L., Mol.Biotechnol., 1995, Jun; 3(3):207-20; Carroll MW., Moss B.;Curr.Opin.Biotechnol., 1997 Oct.; 8(5):573-7; Wojcik J., Acta.Microbiol.Pol., 1995, 44(2):191-6; Ramirez JC. et al., J.Virol., 2000 Aug.; 74(16): 7651-5; Hagen , Anna, et al., Biotechnol.Prog., 1996, 12, 406-408; Huyghe, Bernard, et al., Human Gene Therapy, 1995 Nov., 6:1403-1416).

Im Rahmen der vorliegenden Erfindung sind auch Verfahren zur Vermehrung solcher Viren umfasst, bei denen das virale Genom durch Addition oder Substitution von Sequenzen so verändert wurde, dass das Genom für ein heterologes, also ursprünglich nicht zu dem Virus gehörendes, funktionales Protein mit einer Größe von mindestens 10 kDa kodiert. Erfindungsgemäß wird dabei ein Protein als ein funktionales Protein bezeichnet, wenn das Protein mindestens in der Lage ist, eine Immunreaktion gegen dieses Protein auszulösen. Selbstverständlich kann das Protein neben der immunologischen Aktivität weitere biologische Aktivitäten aufweisen, beispielsweise als Enzym oder Zytokin wirken.

Die Viren, die in den erfindungsgemäßen Verfahren eingesetzt werden, können ferner Deletionen einzelner Gene im viralen Genom aufweisen. So können beispielsweise Gene eines Virus, das als Impfstoff verwendet werden soll, welche für Pathogenitätsfaktoren kodieren, gezielt deletiert werden. Entsprechende Deletionen umfassen vorzugsweise nicht mehr als 500 oder 1000 Nukleotide.

Selbstverständlich kann das für die erfindungsgemäßen Verfahren eingesetzte Virus auch ein vollständiges virales Genom umfassen.

Die Vermehrung der Viren in der Suspensionskultur kann gemäß den erfindungsgemäßen Verfahren in Gegenwart oder Abwesenheit von Serum im Medium erfolgen. Besondere Vorteile ergeben sich durch die Abwesenheit von Serum, da diese Zellkulturbedingungen die Zulassung der medizinischen Verwendung so erzeugter Produkte wesentlich vereinfacht. Durch Verzicht auf Serum-Zusätze zum Kulturmedium werden ferner aufwendige Reinigungsschritte zur Entfernung der Mediumkontaminationen vermieden. Dadurch lassen sich somit auch Verbesserungen hinsichtlich der Qualität des Produktes erreichen und auch Kosten vermeiden.

Als "Serum-freies Medium" wird im Rahmen der vorliegenden Erfindung ein Medium bezeichnet, das keine Zusätze von Serum menschlicher oder tierischer Art aufweist.

Entsprechende Kulturen können definierte Mengen bestimmter Proteine zugesetzt werden, die sich nicht störend auf die Kultur und anschließende Verwendung auswirken. Eine solches Kulturmedium wird als chemisch definiertes Medium bezeichnet.
Diesem Medium werden ausgewählte Proteine, wie mitogene Peptide, Insulin, Transferrin oder Lipoproteine zugesetzt werden, die bei verschiedenen, dem Fachmann bekannten Herstellern bezogen werden können. Unter mitogenen Peptiden werden im Rahmen der vorliegenden Erfindung vorzugsweise pflanzliche Hydrolysate, z.B. Sojabohnen-Proteinhydrolysat oder Lysate aus Proteinen anderer Nutzpflanzen verstanden.

Gemäß einer besonders bevorzugten Ausführungsform sind die Medien jedoch vollständig "Protein-frei". Unter "Protein-frei" werden Kulturen verstanden, bei denen die Vermehrung der Zellen unter Ausschluss von Proteinen, Wachstumsfaktoren, sonstigen Proteinzusätzen und nicht-Serum-Proteinen erfolgt. Selbstverständlich enthalten die in solchen Kulturen wachsenden Zellen selbst Proteine.

Bekannte Serum-freie Medien sind beispielsweise Iscove's Medium, Ultra-CHO-Medium (BioWhittaker) oder EX-CELL (JHR Bioscience). Übliche, Serum-haltige Medien sind beispielsweise Eagle Basalmedium (BME) oder in Minimum Essential Medium (MEM) (Eagle, Science 130, 432, (1959)) oder Dulbecco's Modified Eagle Medium (DMEM oder EDM), die üblicherweise mit bis zu 10% fötalem Kälberserum oder ähnlichen Zusätzen eingesetzt werden. Auch Protein-freie Medien, wie beispielsweise PF-CHO (JHR-Bioscience), chemisch definierte Medien, wie ProCHO 4CDM (Bio Whittaker) oder SMIF 7 (Gibco/BRL-Life Technologies), und mitogene Peptide, wie beispielsweise Primactone, Pepticase oder HyPep^{™} (alle von Quest International) oder Lactalbumin-Hydrolysat (Gibco u.a Hersteller), sind im Stand der Technik hinreichend bekannt. Insbesondere die auf pflanzlichen Hydrolysaten basierenden Medien-Zusätze weisen den besonderen Vorteil auf, dass eine Kontamination mit Viren, Mykoplasmen oder unbekannten infektiösen Agentien ausgeschlossen werden kann.

Gemäß einer bevorzugten Ausführungsform der vorliegenden Erfindung wird während der Kultur der infizierten Zellen frisches Medium, Medium-Konzentrat oder Medienbestandteile wie beispielsweise Aminosäuren, Vitamine, Lipidfraktionen oder Phosphate zugesetzt.

Das erfindungsgemäße Verfahren kann dabei in einem Perfusionssystem oder einem Batch-System durchgeführt werden. Als "Perfusionssysteme" werden Kultursysteme bezeichnet, bei denen kontinuierlich Medium zu- und abgeführt wird. Alternativ dazu können die Zellen auch in einem "Batch-System" kultiviert werden, bei dem das System als weitgehend geschlossenes System ohne Zufuhr von Medium von der Inokulation bis zur Ernte gefahren wird.

Die für die gewünschte Anwendung zu verwendenden Zellkulturbedingungen (Temperatur, Zelldichte, pH-Wert, etc.) sind aufgrund der Eignung der erfindungsgemäß verwendeten Zelllinie über einen sehr weiten Bereich variierbar und können den Bedürfnissen der Anwendung angepaßt werden. Die folgenden Angaben stellen daher lediglich Richtwerte dar.

Die Vermehrung der Zellen vor der Infektion kann ausgehend von Saatkulturen oder kleinen Kulturgefäßen in einem Perfusionssystem unter Verwendung üblicher Rückhalteverfahren, wie beispielsweise Zentrifugation oder Filtration, durchgeführt werden. Es hat sich als vorteilhaft erwiesen, das Kulturmedium bei der Anzucht der Zellen in einem solchen System mit einer Rate von bis zu 3 Fermenterfüllungen pro Tag auszutauschen. Die Zellen können sich unter diesen Bedingungen bis zu Zelldichten von beispielsweise 2 x 10⁷ vermehrt werden. Die Kontrolle der Perfusionsrate erfolgt während der Kultivierung bevorzugt anhand von dem Fachmann bekannten Parametern, wie beispielsweise Zellzahl, Glutamin-, Glucose- oder Lactatgehalt.

Bei Verwendung eines Batch-Systems lassen sich beispielsweise bei einer Temperatur von 37°C und einer Generationszeit von 20-30 Stunden Zelldichten bis etwa 8-25 x 10⁵ Zellen/ml erreichen.

Darüber hinaus lassen sich die Zellen vor der Infektion erfindungsgemäß auch in einem Fed-Batch-System vermehren. Als "Fed-Batch-System" wird im Rahmen der vorliegenden Erfindung ein Kultur-System bezeichnet, bei dem die Zellen zunächst in einem Batch-System angezogen werden, und das Erschöpfen der Nährstoffe (oder eines Teils der Nährstoffe) im Medium durch eine geregelte Zufütterung konzentrierter Nährstoffe kompensiert wird. In einem Fed-Batch-System können die Zellen beispielsweise bis zu einer Zelldichte von etwa 1-10 x 10⁶ vermehrt werden.

Es hat sich weiterhin als vorteilhaft erwiesen, den pH-Wert des Mediums bei der Vermehrung der Zellen vor der Infektion auf einen Wert zwischen pH 6,6 und pH 7,8, und besonders bevorzugt auf einen Wert zwischen pH 7,2 und pH 7,3 einzustellen.

Die Kultur der Zellen vor der Infektion erfolgt bevorzugt bei einer Temperatur zwischen 30° und 40°C, und besonders bevorzugt bei einer Temperatur zwischen 33° und 37°C. Der Sauerstoff-Partialdruck wird bei der Kultur vor der Infektion bevorzugt auf einen Wert zwischen 25% und 95%, und besonders bevorzugt auf einen Wert zwischen 35% und 60% eingestellt. Die im Rahmen der Erfindung angegebenen Werte des Sauerstoff-Partialdrucks basieren auf der Sättigung der Luft.

Für das erfindungsgemäße Verfahren hat es sich als vorteilhaft erwiesen, dass die Infektion der Zellen bei einer Zelldichte von vorzugsweise etwa 8-25x10⁵ Zellen/ml im Batch-System bzw. vorzugsweise etwa 5-20x10⁶ Zellen/ml im Perfusionssystem erfolgt. Die Zellen können in den erfindungsgemäßen Verfahren mit einer viralen Dosis (m.o.i. Wert, "multiplicity of infection"; entspricht der Anzahl an Viruseinheiten pro Zelle zum Zeitpunkt der Infektion) zwischen 10⁻⁸ und 10, bevorzugt zwischen 0,0001 und 0,5, infiziert werden.

Die Kultur der Zellen nach der Infektion kann ebenfalls im Perfusions-, Batch oder Fed-Batch-System erfolgen. Dabei können dieselben Kulturbedingungen wie zuvor eingesetzt werden (Temperatur zwischen 30° und 40°C, Sauerstoff-Partialdruck zwischen 5% und 100%, pH-Wert des Mediums zwischen pH 6,6 und pH 7,8).

Erfindungsgemäß werden ferner Verfahren zur Verfügung gestellt, die eine Ernte und Isolierung der Viren oder der von diesen erzeugten Proteine umfassen. Bei der Isolierung der Viren oder der Proteine werden die Zellen durch Standardmethoden, wie beispielsweise durch Separation, Filtration oder Ultrafiltration, vom Kulturmedium abgetrennt. Im folgenden werden die Viren oder die Proteine nach dem Fachmann hinreichend bekannten Verfahren, wie z.B. Gradienten-Zentrifugation, Filtration, Fällung, Chromatographie, u.ä., aufkonzentriert und anschließend gereinigt. Erfindungsgemäß ist es ferner bevorzugt, dass die Viren während oder nach der Reinigung inaktiviert werden. Eine Virusinaktivierung beispielsweise durch β-Propiolacton oder durch Formaldehyd kann an beliebiger Stelle innerhalb des Reinigungsprozesses erfolgen.

Die erfindungsgemäßen Verfahren eignen sich in besonderer Weise zur Herstellung von Arzneimitteln, insbesondere zur Herstellung von Impfstoffen, und von Diagnosemitteln.

Die Herstellung des Arzneimittels kann dabei die Vermehrung und Isolierung eines Virus oder von diesem erzeugten Proteins und die Vermischung mit einem geeigneten Adjuvanz, Hilfsstoff, Puffer, Verdünnungsmittels und/oder Arzneimittelträger umfassen. Unter Adjuvanzien werden in Rahmen der vorliegenden Erfindung Substanzen verstanden, die die Immunantwort erhöhen. Dazu zählen beispielsweise Hydroxide verschiedener Metalle, wie Aluminiumhydroxid, Bestandteile der bakteriellen Zellwand, Öle oder Saponine. Die Impfstoffe eignen sich insbesondere zur prophylaktischen oder therapeutischen Behandlungen viraler Infektionen.

Die Immunogenität und/oder Wirksamkeit der entsprechenden Impfstoffe kann durch dem Fachmann bekannte Methoden, wie z.B. Schutzversuche mit Belastungsinfektion oder Bestimmung des zur Neutralisation notwendigen Antikörper-Titers festgestellt werden. Die Bestimmung der Virus-Menge bzw. der Menge an produzierten Antikörpern kann beispielsweise durch Bestimmung des Titers oder der Menge an Antigen gemäß dem Fachmann hinreichend bekannten Standardverfahren, wie z.B. Virusttitration, Hämagglutinationstest, Antigenbestimmung oder Proteinbestimmung verschiedenster Art, erfolgen.

Die erfindungsgemäßen Verfahren eignen sich weiterhin zur Herstellung einer diagnostischen Zusammensetzung. Die Zusammensetzungen können einen durch die Verfahren erhältliches Virus oder ein davon erzeugtes Protein umfassen. In Kombination mit im Stand der Technik üblichen Zusätzen und Nachweis-Reagenzien lassen sich diese Zusammensetzungen als diagnostischen Test verwenden, die zum Virus- bzw. Anti-Virus-Antikörper-Nachweis geeignet sind.

In den folgenden Beispielen wurden alle Virustiter nach der dem Fachmann bekannten Endverdünnungsmethode und statistischer 50%-Endpunktbestimmung nach Spearman-Kaerber bestimmt (vgl. Horzinek, Kompendium der allgemeinen Virologie, 2. Auflage, 1985, Parey Verlag, Seiten 22-33). Es wurden jeweils 8 Testkulturen in Mikrotiterplatten mit 100µl-Mengen einer Virusverdünnung infiziert, wobei Verdünnungen des Virusmaterials von 10⁻¹ bis 10⁻⁸ benutzt wurden. Die Auswertung der Virustitrationen erfolgte entweder mikroskopisch anhand des cytopathischen Effektes der Testkulturen oder mit Hilfe von immunologischen Nachweismethoden unter Verwendung virusspezifischer Antikörper.
Die Bindung der virusspezifischen Antikörper wurde als Immunfloureszenz mit Floureszin-markierten Antikörpern oder unter Verwendung von Biotin-markierten Sekundär-Antikörpern und einem Streptavidin/Biotin/Peroxidase-Verstärkerkomplex sowie einem präzipitierbaren Farbstoff (Gregersen et al., Med. Microbiol. Immunol., 177: 91-100) sichtbar gemacht. Die Einheit der Virustiter lautet Kultur-infektiöse Dosis 50% (KID₅₀). Die jeweils für die verschiedenen Virusarten verwendeten virusspezifischen Nachweiszellen und - soweit zutreffend - immunologischen Nachweisverfahren sind in den viursspezifischen Beispielen genannt.

### Beispiele

### Beispiel 1: Handhabung des Zellkultursystem als Suspensionskultur in frühen Arbeitsstufen und im Labormaßstab

MDCK-Zellen aus Saatzellampullen, die in flüssigem Stickstoff gelagert werden, wurden durch Eintauchen in ein Wasserbad schnell aufgetaut und sofort in Kulturmedium (Ultra CHO mit Supplement, BioWhittaker, "Standardmedium") auf eine Zellzahl von etwa 1 x 10⁵ Zellen /ml in der Regel etwa 1:100 - verdünnt. Danach wurden die Zellen durch Zentrifugation (10 Minuten bei 800 x g) von dem Medium getrennt, erneut in frischem Medium aufgenommen und in Spinnerkulturflaschen (100 ml Arbeitsvolumen, Bellco oder Techne) gefüllt. Diese Kulturansätze wurden bei 37°C auf einem Magnetrührer mit 50-60 Umdrehungen/Minute inkubiert. Das Zellwachstum wurde durch Kontrolle der Zellzahl überwacht. Bei Erreichen von Zellzahlen von 8 x 10⁵ bis maximal 1, 6 x 10⁶ Zellen/ml, wurden die Kulturen durch Verdünnung der Zellen in frischem Standardmedium und Aussaat in neue Spinnerkulturflaschen von 100 bis 1000 ml Arbeitsvolumen umgesetzt und bis zur Erreichung maximaler oder gewünschter Zelldichten unter Rühren wie oben beschrieben inkubiert. Bei diesen Zellpassagen wurden die Verdünnung der jeweiligen Kultur im Bereich zwischen 1:4 und 1:10 in der Art an das Zellwachstum angepasst, dass die maximale Zellzahl je nach Bedarf innerhalb von 3 bis 5 Tagen erreicht wurde. Alternativ wurde dieselbe Art der Zellkultivierung ohne Zusatz der Supplemente zum Medium erprobt und konnte über mindestens 10 Passagen problemlos beibehalten werden.

### Beispiel 2: Handhabung des Zellkultursystems als adhärente Kultur

Etablierte Suspensionskulturen (vgl. Beispiel 1) wurden in verschiedenen Medien verdünnt, so dass die Zellzahl etwa 1 x 10⁵ Zellen/ml betrug, und anschließend in verschiedenste Zellkulturgefäße (siehe Tabelle 1) gefüllt. Das Zellkulturvolumen entsprach dabei den für die jeweiligen Kulturgefäße üblichen Mengen, d.h. etwa 4 mm Kulturmedium über der Aussaatfläche bzw. etwa 1 ml Medium pro 2,5 cm² Kulturfläche. Die Kulturen wurden in der Regel bei der für die meisten Zellkulturen üblichen Temperatur von 37°C inkubiert, jedoch waren auch erhebliche Abweichungen der Inkubationstemperatur ohne nennenswerten Verlust möglich (siehe Tabelle 1). Die erprobten Kultursysteme sowie die damit erzielten Ergebnisse des Zellwachstums sind in Tabelle 1 dargestellt und zeigen, dass sich dieses Zellsystem in verschiedenen Medien und Kultursystemen annähernd gleich und robust verhält.

Derartig hergestellte Monolayer-Kulturen wurden für die Titration von Virusernten in Mikrotiterplatten sowie zur Anzucht von Viren unter mikroskopischer Kontrolle oder für Immunfluoreszenz-Untersuchungen, Hämadsorptionstests und andere virologische oder immunologische Standardtmethoden verwendet, die sich besser in adhärenten Einschicht-Kulturen als in Suspensionskulturen durchführen lassen. Des Weiteren waren solche Kulturen besonders geignet, reine Virusstämme durch Plaquereinigung oder Ausverdünnung zu gewinnen. Schließlich wurden die adhärenten Kulturen auch zur Virusvermehrung in kleinen und großen Maßstäben verwendet; größere Mengen dabei vorzugsweise in Rollerflaschen.

**Tabelle 1: Zellwachstum in verschiedenen, adhärenten Kultursystemen**

| Zellkultur System | Zellaussat (× 10⁵ Zellen/ml) | Verwendete Medien | Verwendete Zusätze | Inkubation # | konfluente Kultur nach .. Tagen (8-20 × 105 Zellen/ml) |
|---|---|---|---|---|---|
| Plastik-Kul- | 0,8-1,0 | MEM, | 1-5% FCS | 33 oder 37°C | 4-5 |
| turflaschen | | EDM, | oder | | |
| | | Opti-MEM*, | Supp.* | | |
| | | Ultra CHO*, | | | |
| Plastik-Kul- | 2,0 | MEM, | 1-5% FCS | 33 oder 37°C | 2-3 |
| turflaschen | | EDM, | oder | | |
| | | Opti-MEM*, | Supp.* | | |
| | | Ultra CHO*, | | | |
| Mikrotiter- | 2,0-4,0 | MEM, | 0,5-3% FCS | 33 oder 37°C | 1-2 |
| platten | | EDM, | oder Suppl.* | | |
| | | Opti-MEM*, | | | |
| | | Ultra CHO* | | | |
| Mikrotiter- | 2,0-4,0 | MEM, | 1 % FCS für 1 | 37°C | 1 |
| platten | | EDM, | Tag, dann | | |
| | | Opti-MEM*, | ohne Zusätze | | |
| | | Ultra CHO*, | | | |
| Roller- | 1,0 | EDM, | 0,5-3% FCS | 33 oder 37°C | 4-5 |
| flaschen | | Opti-MEM*, | oder Supp.* | | |
| | | Ultra CHO*, | | | |
| Roller- | 1,0 | EDM, | 1% FCS | 33 oder 37°C | 5-7 |
| flaschen | | Opti-MEM*, | oder Supp.* | | |
| | | Ultra CHO*, | für 3 Tage, | | |
| | | | danach ohne | | |
| | | | Zusätze | | |
| Spinner | 2,0 | BME | 0,5-3% FCS | 33 oder 37°C | 5-7 |
| + Micro- | | MEM | oder Supp.* | | |
| carrier | | EDM | | | |

| | | | | | |
|---|---|---|---|---|---|
| BME: Basal Medium Eagle; Bicarbonat-Ergänzung (2-2,5% einer 5% Stammlösung) MEM: Minimum Essential Medium; Bicarbonat-Ergänzung (2-2,5% einer 5% Stammlösung) EDM: Dulbecco's Modified Eagle Medium; Bicarbonat-Ergänzung (2-2,5% einer 5% Stammlösung) FCS: Fötales Kälberserum Supp.: Ultra CHO supplement #): eingestellte Werte; tatsächlich gemessene Werte mit Abweichungen um +2°Cund -3°C *): Hersteller: Bio-Whittaker | | | | | |

### Beispiel 3: Virusisolierung, Gewinnung und Herstellung von Saatviruspräparationen

Primärisolate, wie beispielsweise virushaltige Organ-, Gewebe-, oder Gewebsflüssigkeitsproben, Rachenabstriche oder Stuhlproben wurden im Eisbad in Standardmedium (beliebige andere Medien oder Phosphatpuffer sind ebenso möglich) mit Antibiotikazusatz (PSN: 100 Einheiten/ml Penicillin, 100 µg/ml Streptomycin, 50 µg/ml Neomycin) aufgeschwemmt und gegebenenfalls homogenisiert (mit Mörsern, Skalpellklingen oder einem sogenannten "Douncer" oder " Potter" Homogenizer fein zerkleinert) . Die erhaltene Suspension wurden mit Hilfe einem laborüblichen Spritzenvorsatzfilter mit einer Porengrösse von 0,45 µm filtriert (zur Isolation kleinerer, unbehüllter Viren auch 0,2 µm). Das Filtrat wurde in kleine Kulturflaschen (25 cm², siehe Beispiel 2) mit frischem Kulturmedium und Antibiotikazusatz inokuliert. Um die Ausbeute zu erhöhen, wurden mehrere Kulturen mit einem Inokulum von beispielsweise 100 µl bis 1 ml versehen und anschließend bei 37°C inkubiert. Für Virusisolate aus den oberen Atemwegen empfiehlt es sich, zusätzlich Kulturen bei einer niedrigeren Inkubationstemperatur von 33°C angesetzt.

Bereits in Kultur vermehrte, reine Virusisolate wurden zur Infektion direkt in dem erfindungsgemäßen Kultursystem gemäß Beispiel 1 oder 2 eingesetzt. Da hierbei jedoch in der Regel ein hoher Virusgehalt der Viruspräparation vorausgesetzt werden konnte, wurden kleinere Inokulummengen von 100 µl oder weniger verwendet. Für derartige Erstinfektionen in dem erfindungsgemäßen Kultursystem wurde eine MOI (multiplicity of infection) von 0,1 und 0,01 bevorzugt; bei unbefriedigendem Ergebnis wurde die Infektion mit MOIs in absteigenden 10er Stufen von 10 bis 0,0001 wiederholt.

Die infizierten Kulturen wurden anschließend täglich mikroskopisch auf virusbedingte Zellschädigungen (CPE, cytopathischer Effekt) untersucht und mit im Kontrollkulturen verglichen. Alternativ sowie bei Viren, die keinen CPE verursachen, wurde die Kultur auf die Anwesenheit bestimmter Virusantigenen oder deren Gene untersucht (z.B. je nach Virusart spezifische HA-Tests; ELISA, PCR). Nach 3 bis 4 Tagen bzw. positivem Befund (Schrumpfung der Zellen, Zelltod, Abrundung und Auflösung des Zellrasens bei adhärenten Kulturen, Plaquebildung) wurden zellfrei zentrifugierte Kulturüberstände als Probe eingefroren, bei negativem oder zweifelhaftem Befund hingegen wurde die gesamte Kultur mit frischem Medium auf eine Zellzahl von 1 x 10 ⁵ Zellen eingestellt (Verdünnung der Suspensionskulturen bzw. Trypsinbehandlung der adhärenten Kulturen mit anschließender Verdünnung der vereinzelten Zellen) und auf neue Kulturen verteilt weiter inkubiert. Da dies bei den meisten Medien einer Verdünnung der Kulturen von 1:4 bis 1:20 entsprach, wurde zur Vermeidung einer logarithmischen Vermehrung der Zahl der Kulturen spätestens nach der zweiten derartigen Kulturpassage nur ein Teil der möglichen Kulturen weiter unterhalten. Nach 3-4 Passagen konnten in der Regel aus geeignetem, virushaltigem Ausgangsmaterial Virusisolate erfolgreich isoliert und nachgewiesen werden.

Für die meisten Virusarten wurde je nach Virusgehalt und Güte des Ausgangsmaterials nach 2-7 tägiger Inkubation ein virusbedingter CPE festgestellt (siehe auch virusspezifische Beispiele). Einige Virusarten vermehren sich jedoch sehr langsam oder zeigen keinen CPE und müssen daher durch verlängerte Passagen und Inkubationsdauer oder spezifische Tests nachgewiesen werden (die jeweils erforderlichen Methoden sind unter den spezifischen Virusbeispielen aufgeführt) . Als Beispiel für ein Virus ohne CPE mit langsamer Vermehrung, welches zudem ein besonderes Nachweissystem erfordert, wird auf das spezielle Beispiel des Hepatitis A-Virus verwiesen. Der dabei beschriebene Nachweistest eignet sich bei Verwendung entsprechender Antiseren ebenfalls zum Nachweis anderer Viren, insbesondere solcher ohne spezifischen CPE.

Ein neu isoliertes Virus sollte zweckmäßigerweise erst nach dreimaliger Plaquereinigung bzw. Herstellung eines reinen Isolates durch die sogenannte "limited dilution" Technik weiter verwendet werden. Die hierzu erforderliche Methoden entsprechend dem Stand der Technik sind einschlägigen Lehrbüchern zu entnehmen (siehe z.B. B.W. Mahy: Virology-a practical approach; IRL Press, Oxford, 1985).

Liegen nun geeignete Viruspräparationen aus einem Primärisolat oder als etablierter Stamm vor, so werden diese anschließend zur Infektion von Spinnerkulturen verwendet, um homogenes Saatvirus für Produktionszwecke zu gewinnen. Ohne den Erfindungsgegenstand darauf zu beschränken, wird empfohlen zunächst eine erste Infektion in kleinen Spinnerkulturen mit 100 ml Kulturmedium vorzunehmen mit MOIs von 10 bis 0,00001, vorzugsweise 0,1 bis 0,0001 zu erproben. Die günstigsten Bedingungen (insbesondere bezüglich MOIs und Erntezeiten) zur Erzielung schneller und hoher Virus titer bzw. Ausbeuten wurden davon ausgewählt, um Saatvirus in einem Kultursystem der erforderlichen Größe - je nach vorgesehenem Produktionsmaßstab und Zahl der Produktionsläufe - in einer weiteren Viruspassage herzustellen. Je nach erzielten Virusausbeuten und vorgesehener Produktionsgröße konnte der Maßstab für diese Saatviruspassage von einigen Spinnerkulturen im bis zu 1000 ml-Maßstab bis hin zu kleineren Fermentern bis hin zu etwa 10 Litern Volumen oder mehr betragen. Das geerntete Virus wurde durch Filtration oder Zentrifugation von eventuellen Zellresten befreit und in produktionsgerechten Kleinmengen aliquotiert bei Temperaturen möglichst unter -70°C gelagert.

### Beispiel 4: Handhabung des Systems als adhärente Mikrocarrier-Kultur für Produktionszwecke

Die Anzucht der adhärenten MDCK-Zellen erfolgte in Rollerflaschen gemäß Beispiel 2, Tabelle 1 mit BME plus 3% fötalem Kälberserum (FCS). Nach der Anzucht in diesem System wurden die Zellen von der Oberfläche der Rollflaschen abgelöst.

Dies geschah enzymatisch mit Hilfe einer geeigneten Trypsinlösung mit üblichen, dem Fachmann bekannten Verfahren. Alternativ wurden gemäß Beispiel 1 Suspensionszellen in Spinnerkulturen angezüchtet und direkt zur Belegung der Microcarrier verwendet werden.

Der Produktionsfermenter wurde mit Microcarriern vom Typ Cytodex 3 (Pharmacia) gefüllt. Die Microcarrier (spezifische Einwaage 5 g/l) wurden autoklaviert und mit Nährmedium konditioniert. Das Verfahren gewährleistete ein Anheften der Zellen an die Oberfläche der Microcarrier. Die auf die oben beschriebene Weise gewonnenen Zellen wurden in das Produktionssystem überführt, sodass die Zelldichte 1x10⁵ Zellen/ml betrug. Die Zellen hafteten an den Microcarriern und wurden bis zur Konfluenz bzw. bis zum Erreichen einer Zelldichte von 3x10⁶ Zellen/ml kultiviert.

Nach der Zellanzuchtphase wurde das vorhandene Nährmedium gegen frisches Nährmedium ausgetauscht. Dazu wurde proteinfreies Nährmedien eingesetzt. Es wurden 2 Waschzyklen gefahren.
Ein Waschzyklus bestand aus dem Abschalten des Rührwerks, dem Absetzen der Microcarrier, der Entnahme des verbrauchten Nährmediums, der Zugabe des frischen Nährmediums und dem Resuspendieren der Microcarrier. Nach dem Waschschritt wurde die Zellkultur mit Trypsin (2,5 mg/l) versetzt.
Anschließend erfolgte die Infektion der Zellkultur mit Saatvirus. Dieses Saatvirus wurde gemäß Beispiel 3 gewonnen und eingesetzt. Die MOI war dabei Virus-spezifisch und betrug zwischen 0,1 und 0,000001 vorzugsweise zwischen 0,01 bis 0,0001. Nach Beendigung der Infektionsphase, deren Dauer einerseits durch das spezifische Virus (siehe spezifische Beispiele), andererseits auch durch die gewählte MOI bestimmt wird, wurde das Rührwerk stillgelegt und die Microcarrier sedimentieren. Der virushaltige Überstand wurde abgezogen und mittels geeigneter Abtrennverfahren von Zellresten gereinigt. Zur Zellabtrennung wurden übliche, dem Fachmann bekannte Zentrifugen bzw. Separatoren, Filter und Querstromfiltrationsanlagen eingesetzt.

### Beispiel 5: Handhabung des Systems als Suspensionskultur bis zum Produktionsvolumen im 1000 1 Maßstab unter Verwendung von serumfreiem Medium

Die Anzucht von Suspensionskulturen für ein Produktionsvolumen von 1000 1 erfolgte mit Hilfe von Spinnerflaschen (Firma Techne) im kleinen Maßstab bis 1000 ml Kulturvolumen (siehe Beispiel 1). Die Zelldichte beim Anlegen der Spinner betrug 1x10⁵ Zellen/ml. Die Zellen wurden im Batchverfahren angezogen und bei einer Zelldichte von 1x10⁶ Zellen/ml durch einfaches Verdünnnen in frischem Medium im Verhältnis 1:10 umgesetzt. Als Medium wurde für die Zellanzucht serumfreies Medium (UltraCHO, Biowhittaker) benutzt. Ab einem Volumen von 10 1 wurden gerührte Fermenter (30 Rührerumdrehungen pro Minute) mit permanenter Belüftung und Kontrolle von Temperatur (Regeltemperatur 37°C für die Zellanzucht), pH-Wert (Regelbereich 7,1 bis 7,3) und Sauerstoff-partialdruck (45 bis 55% pO₂) eingesetzt (technische Details wie in Tabelle 2). Entsprechend einem Umsetzungsverhälnis von 1:10 betrugen die Scale-up Volumina 10 1, 100 1, 1000 1. Die Fermenter erreichten bei einer Anfangszelldichte von 1x10⁵ Zellen/ml die Endzelldichte von 1x10⁶ Zellen/ml in einer Zeit von 3-4 Tagen. Im Maßstab von 1000 1 wurde zusätzlich ein Fed-Batch mit Glucoselösung (100-200 g/l) durchgeführt, um die Zelldichte auf 3x10⁶ Zellen/ml zu steigern. Die erzielten Zellausbeuten sind vergleichend in Tabelle 2 dargestellt.

### Beispiel 6: Handhabung des Systems als Suspensionskultur bis zum Produktionsvolumen bis zu einem Volumen von 1000 l unter Verwendung von chemisch definiertem Medium.

Die Anzucht von Suspensionskulturen für ein Produktionsvolumen von 1000 1 erfolgte analog wie in Beispiel 5 beschrieben. Als Medium wurde hingegen alternativ chemisch definiertes Medium (ProCHO4CDM) für die Zellanzucht benutzt. Es erwies sich als vorteilhaft, 3 bis 5 Vorpassagen zur Adaptation in diesem Medium durchzuführen. Die erzielten Zellausbeuten sind vergleichend in Tabelle 2 dargestellt.

### Beispiel. 7: Handhabung des Systems als Suspensionskultur bis zum Produktionsvolumen im 1000 1 Maßstab unter Verwendung von proteinfreiem Medium

Die Anzucht von Suspensionskulturen für ein Produktionsvolumen von 1000 1 erfolgte analog wie in Beispiel 5 beschrieben. Als Medium wurde proteinfreies Medium (SMIF7, LifeTechnologies) für die Zellanzucht benutzt. Es erwies sich als vorteilhaft, 5-10 Vorpassagen zur Adaptation in diesem Medium durchzuführen.
Die erzielten Zellausbeuten sind vergleichend in Tabelle 2 dargestellt.

**Tabelle 2: Anzucht von Zellen (MDCK 33016) zum Produktionsmasstab im Fermenter unter Anwendung verschiedener Verfahren und Medien**

| Nr. | Verfahren | Medium | N/T/pO2/pH | X₀ | x |
|---|---|---|---|---|---|
| 1 | Batch | UltraCHO | 30 min⁻¹ | 1 × 10⁵ ml⁻¹ | 1 × 10⁶ ml⁻¹ |
| | | | 37°C | | |
| | | | 45-55 % | | |
| | | | 7,1-7,3 | | |
| 2 | Fed-Batch | UltraCHO | 30 min⁻¹ | 1 × 10⁵ ml⁻¹ | 3,1 × 10⁶ ml⁻¹ |
| | | | 37°C | | |
| | | | 45-55% | | |
| | | | 7,1-7,3 | | |
| 3 | Batch | ProCHO4CDM | 30 min⁻¹ | 1 × 10⁵ ml⁻¹ | 1 × 10⁶ ml⁻¹ |
| | | | 37°C | | |
| | | | 45-55% | | |
| | | | 7,1-7,3 | | |
| 4 | Fed-Batch | ProCHO4CDM | 30 min⁻¹ | 1 × 10⁵ ml⁻¹ | 3,3 × 10⁶ ml⁻¹ |
| | | | 37°C | | |
| | | | 45-55% | | |
| | | | 7,1-7,3 | | |
| 5 | Batch | SMIF7 | 30 min⁻¹ | 1 × 10⁵ ml⁻¹ | 1 × 10⁶ ml⁻¹ |
| | | | 37°C | | |
| | | | 45-55 % | | |
| | | | 7,1-7,3 | | |
| 6 | Fed-Batch | SMIF7 | 30 mid⁻¹ | 1 × 10⁵ ml⁻¹ | 3,0 × 10⁶ ml⁻¹ |
| | | | 37°C | | |
| | | | 45-55 % | | |
| | | | 7,1-7,3 | | |

| | | | | | |
|---|---|---|---|---|---|
| X₀: Anfangszelldichte X: Endzelldichte N/T/pO₂/pH: Rührerumdrehnungen, Temperatur, Sauerstoff-Partialdruck, pH-Wert | | | | | |

### Beispiel 8: Handhabung des Systems in der Produktionsphase mit serumfreiem Medium

Nach der Anzucht von Suspensionskulturen bis zum Produktionsmaßstab gemäß Beispiel 5 wurden die Zellen auf drei Fermenter gleichen Volumens 3x1000 1 verteilt und mit frischem Medium aufgefüllt. Damit erhielt jeder Fermenter 1/3 Volumen Vorkultur und 2/3 Volumen Frischmedium. Es wurde das gleiche Medium wie in der Anzuchtphase verwendet (UltraCHO, BioWhittaker). Nach dem Auffüllen wurde die Zellkultur mit Trypsin 10 mg/l versetzt. Anschließend erfolgte die Infektion der Zellkultur mit einem Saatvirus (Influenza B/Harbin/7/94) bei einer MOI von 0,001 und eine weitere Inkubation unter gleichen Fermentationsbedingungen wie bei der Zellanzucht, jedoch bei 33°C, über 96 Stunden. Der zellhaltige Überstand wurde im folgenden abgezogen, und die Zellen wurden dabei mittels eines Separators abgetrennt. Es erfolgte ein weiterer Filtrationsschritt durch Kartuschenfilter mit einer Porengröße von 0,45 µm zur Abtrennung weiterer Feinpartikel.

Die Virusernten wurden auf ihren Virusgehalt mit Hilfe von Standardmethoden im HA-Test mit 0,5% Hühnererythrozyten und durch Virustitration in adhärenten MDCK-Zellen getestet: Der gemessene HA-Gehalt betrug 1024 Einheiten, der Virustiter lag bei 10^{8,2} KID₅₀/ml.

### Beispiel 9: Handhabung des Systems in der Produktionsphase mit chemisch definierten Medien

Die Vorbereitung der Produktionzellen erfolgte wie in Beispiel 8 beschrieben. Als Frischmedium wurde jedoch chemisch definiertes Medium (ProCHO4CDM, BioWhittaker) verwendet. Nach dem Auffüllen wurde die Zellkultur mit Trypsin 2,5 mg/l versetzt. Die weitere Infektion wurde wie in Beispiel 8 beschrieben durchgeführt.
Der gemessene HA-Gehalt betrug 1024 Einheiten, der Virustiter lag bei 10^{7,5} KID₅₀/ml.

### Beispiel 10: Handhabung des Systems in der Produktionsphase mit proteinfreiem Medium

Die Vorbereitung der Produktionszellen erfolgte wie in Beispiel 8 beschrieben. Als Frischmedium wurde jedoch proteinfreies Medium (SMIF7, Life Technologies) verwendet. Nach dem Auffüllen wurde die Zellkultur mit Trypsin 2,5 mg/l versetzt.
Die weitere Infektion wurde wie in Beispiel 8 beschrieben durchgeführt. Der gemessene HA-Gehalt betrug 1024 Einheiten, der Virustiter lag bei 1.0^{7,9} KID₅₀/ml.

### Beispiel 11: Anzucht und Infektion mit chemisch definiertem Medium

Die Anzucht der Zellen erfolgte wie in Beispiel 6 beschrieben, die Infektion wie in Beispiel 9 beschrieben. Somit erfolgte die gesamte Zellkultur von der Anzucht bis zur Ernte der Infektion in chemisch definiertem Medium.

### Beispiel 12: Anzucht mit chemisch definiertem Medium und Infektion in proteinfreiem Medium

Die Anzucht der Zellen erfolgte wie in Beispiel 6 beschrieben in chemisch definiertem Medium, die Infektion wie in Beispiel 10 beschrieben in proteinfreiem Medium.

### Beispiel 13 : Anzucht und Infektion in proteinfreiem Medium

Die Anzucht der Zellen erfolgte wie in Beispiel 7 beschrieben.

Die Infektion wie in Beispiel 10 beschrieben. Somit erfolgte die gesamte Zellkultur von der Anzucht bis zur Ernte der Infektion in proteinfreiem Medium.

### Beispiel 14: Allgemeine Beschreibung der Virusreinigung

Nach Abschluss der Virusvermehrungsphase wurde die Zellkulturernte durch einen Tiefenfilter mit einer Porengröße von 0,45 oder 0,5 µm filtriert, um Zellen und Zellbruchstücke abzutrennen. Alternativ wurde diese Abtrennung mit Hilfe eines Separators durchgeführt. Die in der geklärten Ernte enthaltenen Viren wurden bei Bedarf mittels Ultrafiltration konzentriert und gereinigt, wobei eine. Membran mit einer Ausschlussgrenze zwischen 50.000 und 1.000.000, vorzugsweise 100.000 bis 500.000 verwendet wurde. Das erhaltene Viruskonzentrat wurde auf eine Chromatographiesäule geladen, welche mit CS (Cellufine Sulfate, Millipore) gepackt war. Nachdem Verunreinigungen durch Waschen mit Puffer entfernt wurden, wurden die Viren mit einer 0,3 bis 3 M Kochsalzlösung eluiert. Das Eluat wurde mittels Ultrafiltration entsalzt und weiter konzentriert. Alternativ oder in Kombination mit einer chromatographischen Reinigung kann ein weiter Reinigungseffekt durch Ultrazentrifugation erzielt werden. Die meisten Virusarten können überdies entsprechend ihrer Schwebedichte duch Ultrazentrifugation in einem Saccharosegradienten mit nachfolgender Fraktionierung des Gradienten gereinigt werden. Eine Virusinaktivierung mit Formaldehyd oder β-Propiolacton kann an beliebiger Stelle innerhalb des Reinigungsprozesses eingefügt werden, wird jedoch vorzugsweise nach der Konzentrierung oder nach der Reinigung eingesetzt, da das zu inaktivierende Volumen dann bereits erheblich reduziert ist.

### Beispiel 15: Gewinnung von inaktivierten, reinen Viruspräparationen zur Formulierung von Impfstoffen

Flaviviren (Virus der Frühsommer-Meningoenzephalitis, Stamm K 23) wurden gemäß Beispiel 5, 6 und 7 in verschiedenen Medien bei einer Animpfdosis von 0,2 MOI angezüchtet (für weitere Details vgl. Beispiel 19).
Das geerntete, virushaltige Kulturmedium wurde durch Zentrifugation und Filtration über Filter mit einer Porengröße von 0,45 µm von eventuell enthaltenen Zellresten befreit. Aus Sicherheitsgründen wurde dieses Material bereits nach der Filtration durch Zusatz von β-Propiolacton in einer Verdünnung von 1:2000 bzw. 1:2500 und Inkubation bei 2-8°C über 24 Stunden inaktiviert. Ein Zellkulturtest der inaktivierten Präparationen nach 2-stündiger Hydrolyse des Inaktivierungsmittels bei 37°C zeigte, dass bis zu einer Nachweisgrenze von weniger als 0,03 infektiösen Einheiten/ml kein aktives Virus mehr enthalten war.

Für die Analytik der nachfolgend beschriebenen Reinigungsschritte wurde zur Bestimmung des Gesamtproteingehaltes ein BCA-Assay (Pierce) benutzt. Der spezifische Antigengehalt wurde mit Hilfe eines Sandwich-ELISA unter Verwendung spezifischer monoklonaler Antikörper gegen das E-Glykoprotein (Niedrig et al., 1994, Acta Virologica 38: 141-149) und eines selbst hergestellten, polyklonalen Antiserums gegen gereinigtes Virus aus Kaninchen bestimmt. Die Werte für das inaktivierte Ausgangsmaterial wurde dabei als Referenzwert (entsprechend 100%) angesetzt.

### Reinigung durch Gradientenzentrifugation:

Inaktivierte Viruspräparationen wurde nach bekannten Methoden über eine Dichtegradienten-Ultrazentrifugation (15-60% Saccharose) bei 80.000 x g gereinigt. Anschließend wurde der Gradient fraktioniert und in Proben der Fraktionen die Extinktion bei 280 nm zur Identifizierung des Viruspeaks bestimmt. Eine starke Erhöhung der Extinktion wurde jeweils im Bereich einer Saccharosekonzentration zwischen 30 und 40% festgestellt, das Maximum lag bei 34 und 35%. In diesem Bereich wurde auch der höchste Gehalt an spezifischem Virusprotein und die höchste Reinheit (bestimmt als Verhältnis von Virusprotein zu Gesamtprotein) gemessen. Insgesamt wurden in diesen Peakfraktionen mehr als 50% des im Ausgangsmaterial festgestellten, spezifischen Antigengehaltes wiedergefunden.

### Chromatographische Reinigung:

Die inaktivierten Viruspräparationen (siehe oben) wurden auf eine CS-Säule aufgetragen, welche zuvor mit 5 Säulenvolumen 50 mM Phosphatpuffer, pH 7,5 äquilibriert wurde. Anschließend wurde mit 10 Säulenvolumen Phosphatpuffer gewaschen, um ungebundenes Material zu entfernen. Gebundenes Material wurde nachfolgend mit demselben Phosphatpuffer unter stufenweiser Zumischung von steigenden Mengen desselben Puffers mit Zusatz von 3 M NaCl eluiert. Im Durchfluss beim Auftragen des Virusmaterials wurden zwischen 3,2 und 3,9% des spezifischen Antigens und 79 bis 83% des Gesamtproteins analytisch wiedergefunden. Im Waschpuffer fand sich 6-11% des Gesamtproteins und 0-2,3% des Antigens. Mehr als 95% des Antigens wurde somit an das Säulenmaterial gebunden.
Bei der Elution mit 0,6 bis 1,8 M NaCl wurden etwa 60,0% des Antigens wiedergefunden, die höchste Reinheit wurde bei einer Elution mit 1,2 M NaCl erzielt. Höhere Salzkonzentrationen bis 3 M NaCl eluierten weitere, geringe Mengen (< 15%) an Antigen mit geringerer spezifischer Reinheit.

Reinigung durch Kombination von Chromatographie und Ultrazentrifugation Vereinigte Eluate nach 0,6 und 1,2 M NaCl-Elution aus einer chromatographischen Reinigung wie oben beschrieben wurden einer Ultrazentrifugation über 2,5 Stunden bei 80 000 x g unterworfen.
Das Viruspellet wurde in 50mM Phosphatpuffer pH 7,5 resuspendiert und analysiert. Die Gesamtproteinkonzentration dieser Präparation war auf 0.7 % des Ausgangsgehaltes reduziert, der Reinheitsgrad war durch diesen Schritt um das Zehnfache erhöht worden.
Diese Viruspräparation wurde einer Gradientenreinigung wie oben beschrieben unterworfen. Es wurde nach Fraktionierung ein sehr ähnliches Gradientenprofil gefunden, wie dies nach direkter Gradientenreinigung erzeilt wurde. Die Spitze des Viruspeaks hatte sich allerdings geringfügig verschoben und lag nun bei 37% Saccharose.

### Beispiel 16: Vermehrung von Flaviviren

Suspensionskulturen der MDCK 33016-Zelle mit einer Zelldichte von 1 - 1,5 x 10⁶ Zellen/ml wurden unter den Standardbedingungen (Standardmedium, 37 °C Kultur- und Tnfektionstemperatur) mit dem Virus der Frühsommer-Meningoencephalitis (Stamm K23, Niedrig et al., 1994, Acta Virologica 38: 141-149) infiziert. Abweichend zu den vorstehenden Beispielen wurden zur Infektion stark variierende MOI's eingesetzt. Darüberhinaus wurden die Infektionskulturen teilweise in chemisch definiertem Medium oder in Medium ohne proteinhaltige Zusätze gehalten. Es wurden verschiedene Kultur- und Ernteverfahren angewandt, die beispielhaft zeigen, dass sich mit System auch bei Änderung verschiedener Parameter hohe Ausbeuten erzielen lassen und sogar mehrfache Ernten möglich sind. Diese Änderungen sind in Tabelle 3 zusammengefasst. Die Virustitrationen erfolgten in A 549-Zellen (ECACC Nr. 86012804) und wurden nach 5 Tagen anhand der CPE ausgewertet. Bemerkenswert war die Tatsache, dass die wiederholte Ernte ein und derselben Kultur jeweils mit einem Austausch des Kulturmediums einherging, so dass die Zellen bei jeder Ernte mit neuem Medium versorgt wurden und daher weiter wachsen konnten. Ohne diese Ernten wäre die Kultur nicht über längeren Zeitraum lebensfähig und produktiv geblieben. Da die häufigen Mediumaustausche in kurzen Abständen die hohe Stoffwechselleistung der Kulturen nicht zu kompensieren vermochte, erfolgten weitere Mediumergänzungen und Vergrößerungen der Kulturen nach 4 bzw. 5 Tagen Infektionszeit.

**Tabelle 3:Vermehrung von FSME Virus/K23 in MDCK 33016-Kulturen in Standardmedium sowie in alternativen Medien unter Verwendung verschiedener MOI und Erntevarianten**

| MOI | | Ausbeute (log 10 KID₅₀/ml) bei Ernte nach ... Tagen | | | | | | Medium | |
|---|---|---|---|---|---|---|---|---|---|
| | 1 | 2 | 3 | 4 | 5 | 6 | 7 | 8 | Verwendetes Medium |
| Ansätze mit Mehrfachemten bei vollständigem Medienausaustausch: | | | | | | | | | |
| 2,0 | | 9,0 | | 8,8 | | | 8,8 | | Standardmedium |
| 2,0 | | | 9,0 | | (M- +30)⁺ | | 8,4 | | Standardmedium |
| 2,0 | | | 6,1 | | (M+30) | | 6,1 | | Proteinfreies Medium |
| 0,2 | | | 7,8 | | (M+30) | | 7,8 | | Chem. definiertes Medium |
| 0,2 | | 8,7 | | 8,0 | | | 7,7 | | Standardmedium |
| 0,2 | | 8,3 | | (M+30) | 8,6 | | | | Standardmedium |
| 0,2 | | | 9,0 | | (M+30) | 9,0 | | | Standardmedium |
| 0,2 | | 8,6 | | 9,2 | | | 9,0 | | Standardmedium |
| 0,2 | | 9,0 | | | 9,0 | | | 8,6 | Standardmedium |
| 0,2 | | | 7,3 | | (M+30) | 8,2 | | | proteinfreies Medium |
| 0,2 | | | 7,2 | | (M+30) | 8,6 | | | Chem. definiertes Medium |

| Ansätze mit Probennahme ohne Medienaustausch oder -Ergänzung | | | | | | | | | |
|---|---|---|---|---|---|---|---|---|---|
| 10^{-0,3} (= 0,5) | 7,7 | 8,3 | 9,2 | 9,4 | 9,3 | | | | Standardmedium |
| 10^{-0,3} | 6,3 | 7,5 | 8,4 | 8,6 | 8,9 | | | | Medium MEM, adhärente Kultur, 1% FCS |
| 10^{-1,3} (=- 0,05) | 5,2 | 6,3 | 6,6 | 6,8 | 6,8 | | | | Standardmedium, Temperaturüberschreitung durch Rührer |
| 10-^{1,3} | 5,1 | 6,2 | 7,1 | 8,0 | 8,4 | | | | Standardmedium |
| 10-^{2,3} | 4,8 | 6,2 | 7,6 | 7,5 | 8,1 | | | | Standardmedium |
| 10^{-3,3} | 3,4 | 4,7 | 4,9 | 5,6 | 6,0 | | | | Standardmedium |
| 10^{-4,3} | 2,7 | 3,7 | 4,3 | 4,3 | 4,4 | | | | Standardmedium |
| 10^{-5,3} | 2,5 | 2,6 | 3,4 | 3,7 | 4,3 | | | | Standardmedium |

| | | | | | | | | | |
|---|---|---|---|---|---|---|---|---|---|
| ⁺⁾(M+30) bedeutet Mediumergänzung +30% des Kulturvolumens am angegebenen Tag | | | | | | | | | |

### Beisipiel 17: Vermehrung von Picornaviren

Adhärente MDCK-33016 Kulturen wurden zur Infektion mit Hepatitis A Virus (HAV, Stamm HM 175, ATCC VR-1358) in MEM-Medium mit Zusatz von 5% fötalem Kälberserum und Bicarbonat (vergleiche Beispiel 2) kultiviert. Im Rahmen des Versuches wurde außerdem ein weiteres Virusisolat "München" verwendet (vergleiche Frösner et al. 1979; Infection 7: 303-305). Das Virus wurde verdünnt in die frisch angesetzte Kultur inokuliert, und die Kulturen wurden bei 37°C inkubiert. In abwechselndem Turnus von 3-4 Tagen wurden die Kulturen 1:4 weiter passagiert.

Suspensionskulturen von MDCK-33016 Zellen wurden in Standardmedium gemäß Beispiel 1 kultiviert, mit HM 175 inokuliert und bei 33°C inkubiert und anschließend wöchentlich 1:10 passagiert. Die adhärenten Zellen und Suspensionskulturen wurden nach Infektion für bis zu 35 Tage weiter unterhalten. Danach erfolgte der Nachweis der aktiven Virusreplikation anhand des CPE (Stamm HM 175) oder nach einer bereits beschriebenen Methode (siehe Virustitration, Seite 93 in Gregersen et al. 1988; Med. Microbiol. Immunol. 177: 91-100). Abweichend wurde als virusspezifischer Antikörper ein humaner Anti-HAV-Antikörper als gereinigtes IgG eingesetzt (Bezeichnung F 86012, freundlicherweise überlassen von der Firma Dade Behring). Als Anti-human IgG Antikörper mit Biotin-Markierung wurde Produktnummer 39015 (Firma Sigma) eingesetzt. Der spezifische Nachweis einer aktiven Virusvermehrung mit diesem System liefert braun-rosa gefärbte Zellen, die bei geringer Vergrößerung im Mikroskop leicht zu erkennen sind. Virus-negative Zellen erscheinen dagegen ungefärbt oder weisen nur eine ganz geringfügige Färbung auf. Mit denselben Nachweismethoden wurden auch Virustitrationen drei Wochen nach dem Ansatz ausgewertet, für die humane diploide Zellen (MRC-5) als Kultursystem benutzt wurden.

In allen oben beschriebenen Infektionsansätzen und mit beiden verwendeten Virusisolaten konnte eine aktive HAV-Replikation in den MDCK-Zellen nachgewiesen werden. In Suspensionskulturen wurde mit dem Stamm HM 175 eine überraschend schnelle Virusvermehrung nachgewiesen. Am Tag 7 nach Infektion lag der gemessene Virustiter im Überstand bei 10^{5,4} KID₅₀/ml; diese Kultur wurde wöchentlich durch einfache Verdünnung 1:10 passagiert und lieferte in den resultierenden Kulturen wiederum nach weiteren 7 Tagen ähnliche Virustiter. Am Ende der Kultivierung und nach zwei weiteren Zellpassagen wurden die Virustiter in einer Probe des zellfreien Medium bestimmt. Zusätzlich wurde eine Probe der gesamten Kultur genommen und die darin enthaltenen Zellen durch zweimaliges Einfrieren bei -20°C und Auftauen aufgeschlossen.
Die Zellbestandteile wurden durch Zentrifugation entfernt, bevor diese Proben titriert wurden. Die erhaltenen Virusausbeuten aus diesem Ansatz sind in der Tabelle 4 zusammengefaßt und zeigen, dass ohne Einbuße der spezifischen Ausbeuten eine wöchentliche Verzehnfachung der Kulturen möglich ist, wobei trotz der massiven Mengenzunahme gute Virustiter pro Volumeneinheit geerntet werden können. Bemerkenswerterweise wird dabei ein beträchtlicher Anteil an Virus im Überstand gefunden, was für dieses stark zellgebundene Virus ebenfalls überraschend ist (siehe Tabelle 4)

**Tabelle 4: Vermehrung von Hepatitis A-Virus (Stamm HM 175) in MDCK 33016 Suspensionskulturen unter stetiger Vermehrung und Vergrößerung des Kulturvolumens.**

| *Tag nach Infektion* | *Zellpassage (Vergößerung des Kulturvolumens)* | *Relatives Erntevolumen (Tag 0* = 1) | *Virus Gesamtausbeute (KID₅₀)* | |
|---|---|---|---|---|
| | | | *im Medium* | *nach Zellaufschluß* |
| *7* | *1:10* | 1 | *10^{7,4}* | *10^{7,8}* |
| *14* | *1:10* | *10* | *10^{8,5}* | *10^{9,2}* |
| *21* | *1:10* | *100* | *n. b.* | *n. b.* |
| *28* | *1:10* | *1000* | *10^{10,8}* | *10^{11,4}* |
| *35* | *Ende* | *10000* | *10^{12,5}* | *10^{14,2}* |

### Beispiel 18: Vermehrung von Rhabdoviren

Suspensionskulturen in Standardmedium gemäß Beispiel 1 wurden in Zellkulturflaschen mit einer Zelldichte von 1 x 10⁶ Zellen pro ml Medium ausgesät. Nach dem Anwachsen der Kulturen wurden jeweils zwei Kulturen mit einer MOI von 0,01 und eine Kultur mit einer MOI von 0,001 mit einem Tollwutvirus (Stamm Pitman-Moore, Impfstoff-Virusstamm) infiziert. Die Kulturen wurden bei 37°C inkubiert und alle 4 bzw. 3 Tage mit Trypsin abgelöst und im Verhältnis 1:10 (nach 4 Tagen) bzw. 1:8 (nach 3 Tagen) passagiert und so 18 Tage lang unterhalten (siehe Tabelle 5). Der Infektionserfolg wurde bei jeder Passage verfolgt. Eine Kultur wurde mit einer 3,5%igen Formalinlösung versehen und drei Tage lang bei Raumtemperatur in dieser Lösung inkubiert, um eine Inaktivierung der Viren zu erreichen. Nach Entfernen der Formalinlösung wurde die Kultur mit PBS gewaschen und 25 Minuten mit 1% Triton-X100 in PBS bei Raumtemperatur inkubiert. Nach Entfernen dieser Lösung wurde dreimal mit PBS gewaschen und ein FITC-markierter Antikörper gegen Tollwutvirus (50 µl 1:400 verdünntes Kaninchen Anti-Tollwut-IgG-FITC, Dade Behring, OSHY 005) aufgetragen. Nach 90 Minuten Inkubation bei 37°C wurde wiederum mit PBS gewaschen und die Kultur unter einem inversem Fluoreszenzmikroskop beurteilt.

Alternativ wurde nach Standardmethoden Virustitrationen der Kulturüberstände in MRC-5 Zellen durchgeführt, die ebenfalls nach Formalin/Triton-Vorbehandlung mittels Immunfluoreszenz wie oben beschrieben ausgewertet wurden. Anhand der mit diesem System erzielten Virustiter wurde eine grobe Korrelation zu den Ausbeuten im entsprechenden Herstellverfahren unter Verwendung von MRC-5 Kulturen für einen zugelassenen Humanimpfstoff (Rabivac) vorgenommen, die eine Orientierung ermöglicht, wieviel Impfstoff-Antigen pro ml Kulturernte enthalten ist (siehe Tabelle 5).

Beide Ansätze (MOI 0,01 und 0,001) zeigten bereits nach 4 Tagen positive Ergebnisse und danach einen ähnlichen Infektionsverlauf, allerdings war bei der geringeren MOI der Infektionsverlauf - erkennbar an den Virustitern, die bis zum Tag 11 etwa 1,2 bis 0,5 log KID₅₀ geringer lagen - geringfügig verlangsamt. Ab der 3. Passage der Kulturen am Tag 11 zeigte sich in allen Kulturen eine sehr intensive, spezifische Immunfluoreszenz mit beginnender Zellzerstörung, die danach weiter zunahm, bis im Verlaufe der 5. Passage am Tag 18 ein Großteil der Zellen völlig zerstört war, so dass die Infektion beendet wurde. Der Gehalt an spezifischem Virus nahm bis zum Tag 14 stetig zu, um danach infolge der zunehmenden Zellzerstörung wieder abzunehmen. Die Ergebnisse dieses Infektionsverlaufes sind in folgender Tabelle zusammengefasst und zeigen, dass - gemessen an der bekannt langsamen Virusvermehrung von Rabiesviren - eine sehr schnelle Virusvermehrung ohne Adaptation in diesen Zellen zu erwarten ist, wobei trotz fortlaufender Weitervermehrung der Zellen in regelmäßigen Abständen und wiederholt gute Antigenausbeuten geerntet werden können.

**Tabelle 5: Vermehrung von Tollwutvirus in MDCK 33016 Kulturen bei fortlaufender Vergrößerung des Kulturvolumens**

| Tag nach Infektion | Passage der Zellen | Relatives Kulturvolumen | Tollwut-Antigen (Impfdosen/ml) |
|---|---|---|---|
| 4 | 1:4 | 1 | nicht bestimmt |
| 7 | 1:3 | 4 | nicht bestimmt |
| 11 | 1:4 | 12 | 0,2-0,4 |
| 14 | 1:3 | 36 | 0,4-0,5 |
| 18 | entfällt | 108 | 0,4-0,5 |

In ähnlicher Weise wurde das gleiche Virus direkt in Suspensionskulturen gemäß Beispiel 1 inokuliert, wobei zusätzlich eine MOI von 0,0001 eingesetzt wurde. Es wurde wiederum ausschließlich Standardmedium für den gesamten Infektionsverlauf eingesetzt und die Kulturen wurden ebenfalls wöchentlich zweimal 1:8 bzw. 1:10 umgesetzt. Das Umsetzen erfolgte hierbei jeweils nur durch einfaches Verdünnung der Zellen in frischen Medium und Neuaussaat. Der Infektionserfolg wurde hierbei nur noch anhand von Virustitrationen in MRC-5 Zellen wie oben beschrieben verfolgt. Die Infektionen lieferten bei allen drei MOIs bereits nach 4 Tagen positive Virustiter im Kulturüberstand. Die Virustiter stiegen nach anfänglichen Verdünnungsverlusten nach dem 7. Tag von Passage zu Passage und trotz der immer wieder durchgeführten, exponentiellen Verdünnung stetig an, führten jedoch in diesen Suspensionskulturen zu keiner massiven Zellzerstörung. Die Infektion wurde bis zur 8. Passage (Tag 28 nach Infektion) verfolgt und dann abgebrochen.

Virusproben aus diesen Infektionen wurden als Saatvirus eingefroren und für eine neue Infektion von Suspensionskulturen, beginnend mit 100 ml und ebenfalls in Standardmedium und unter gleichen Passagebedingungen wie oben beschrieben verwendet. Die MOI wurde in diesem Falle auf 0,000025 reduziert. Die Infektion wurde über 6 Zellpassagen (21 Tage) unterhalten. Bei trotz der massiven Passageverdünnungen langsam steigenden Virustitern wurden am Ende dieses Infektionslaufes Virustiter gemessen, die umgerechnet etwa 0,3 Impfstoffdosen pro ml Kulturüberstand ergaben. Wäre tatsächlich das gesamte Kulturvolumen und nicht jeweils nur ein Teil davon weiterpassagiert worden, hätten nach den 6 Passagen etwa 500 Liter Kultur geerntet werden können, die Virusausbeute hätte dabei etwa 150 000 Impfstoffdosen entsprochen.

### Beispiel 19: Vermehrung von Reoviren

Suspensionskulturen der MDCK 33016-Zelle in Standardmedium wurden mit Reovirus Typ 3 (erhalten von Firma Bio Doc, Hannover) bei einer MOI von 0,01 infiziert und 3 oder 5 Tage bei 33°C oder 37°C weiter inkubiert. Proben der Kulturüberstände wurden nach 5 und 7 Tagen entnommen und in dem überlieferten System unter Verwendung von BHK-Zellen in MEM-Medium mit 3% FCS titriert. Die Auswertung der Titrationen erfolgte nach 7 Tagen.
Die Virusausbeuten der Suspensionskulturen nach 5 Tagen betrugen bei 37°C 10^{8,1} KID₅₀/ml, bei 33°C 1.0^{8,0} KID_{50/}ml. Nach 7 Tagen lagen die Titer bei beiden Temperaturansätzen bei 1.0^{8,0} KID₅₀/ml.

Derselbe Virusstamm wurde unter chemisch definierten und proteinfreien Medien analog zu Beispiel 12 in MDCK-33016 Kulturen zur Infektion bei einer MOI von 0,01 verwendet. An den Infektionstagen 3, 7, und 10 wurden jeweils 22% des Kulturvolumens entnommen und durch frisches Medium ersetzt. Am Tag 7 wurde 50% des Kulturvolumens einschließlich der Zellen entnommen und durch neues Medium ersetzt. Somit wurde insgesamt das Kulturvolumen im Laufe der Infektion fast komplett ausgewechselt und durch Mediumergänzung den Zellen Gelegenheit gegeben, sich entsprechend der Ausverdünnung weiter zu vermehren. Das verwendete Verfahren entspricht insgesamt etwa einer 1:2 Passage der Kultur, wobei lediglich die Mengenüberschüsse entfernt wurden. Die besonders in der Anfangsphase mögliche, erheblich höhere Passage bzw. Ausverdünnung der Kulturen wurde hier bei Weitem nicht voll ausgenutzt.

Folgende Virusausbeuten wurden gemessen:

| | | | | |
|---|---|---|---|---|
| Infektionstag: | 3 | 7 | 10 | 14 |
| log KID₅₀/ml : | 5,4 | 7,1 | 6, 6 | 6,6 |
| (Mittelwerte aus Doppeltestungen | | | | |

## Patentansprüche

1. Verfahren zur Herstellung eines Arznei- oder Diagnosemittels, Schritte umfassend, bei denen man
(a) MDCK-Zellen mit einem Virus infiziert;
(b) nach der Infektion die MDCK-Zellen in Zellkultur unter Bedingungen zieht, die eine Vermehrung der Viren und gleichzeitig eine gezielte weitere, mindestens 2-fache Vermehrung der Zellen durch eine Vergröβerung des gesamten Kulturvolumens ermöglichen,
wobei das Virus ausgewählt ist aus der Gruppe bestehend aus Viren der europäischen Frühsommer-Meningoenzephalitits (FSME) sowie der verwandten östlichen (russischen oder anderen) Formen, Japanisches Enzephalitis-Virus, Dengue Virus, Gelbfiebervirus, Tollwutviren, Hepatitis A Viren, oder Rotaviren.

2. Verfahren nach Anspruch 1, **dadurch gekennzeichnet, dass** die Zellen nach der Infektion unter Bedingungen gezogen werden, die mindestens 5-fache vorzugsweise mindestens eine 10-fache Vermehrung der Zellen bewirken.

3. Verfahren nach Anspruch 2, **dadurch gekennzeichnet, dass** die Zellen nach der Infektion mindestens 7, 21 oder 28 Tage, vorzugsweise mindestens 35 Tage in Zellkultur gezogen werden.

4. Verfahren gemäß einem der Ansprüche 1 bis 3, **dadurch gekennzeichnet, dass** man während der gezielten Vermehrung der Viren und der Zellen mindestens einmal frisches Medium, Medium-Konzentrat oder Medienbestandteile zugibt oder mindestens einen Teil der Viren und Zellen in ein Kulturgefäß überführt, welches frisches Medium, Medium- Konzentrat oder Medienbestandteile enthält.

5. Verfahren gemäß einem der Ansprüche 1 bis 4, **dadurch gekennzeichnet, dass** die Zugabe des Mediums, des Medium-Konzentrates oder der Medienbestandteile oder die Überführung der Zellen und Viren in ein anderes Kulturgefäß mindestens einmal wiederholt wird.

6. Verfahren gemäß einem der Ansprüche 1 bis 5, **dadurch gekennzeichnet, dass** die Zugabe des Mediums, des Medium-Konzentrates oder der Medienbestandteile oder die Überführung der Zellen und Viren in ein anderes Kulturgefäß mehrfach wiederholt wird.

7. Verfahren gemäß einem der Ansprüche 1 bis 6, **dadurch gekennzeichnet, dass** Medium während der Vermehrung der Viren und der Zellen entnommen wird.

8. Verfahren gemäß einem der Ansprüche 1 bis 7, **dadurch gekennzeichnet, dass** während der Vermehrung der Viren und der Zellen kontinuierlich Medium entnommen und frisches Medium, Medium-Konzentrat oder Medienbestandteile zugegeben wird.

9. Verfahren gemäß einem der Ansprüche 1 bis 8, **dadurch gekennzeichnet, dass** die Zellen vor der Infektion adhärent oder als Suspensionskultur gezogen werden.

10. Verfahren gemäß einem der Ansprüche 1 bis 9, **dadurch gekennzeichnet, dass** die Zellen nach der Infektion adhärent oder als Suspensionskultur gezogen werden.

11. Verfahren gemäß einem der Ansprüche 1 bis 10, **dadurch gekennzeichnet, dass** die MDCK-Zellen von der Zelllinie MDCK-33016 abstammen.

12. Verfahren gemäß einem der Ansprüche 1 bis 11, **dadurch gekennzeichnet, dass** das Medium Serum-frei ist.

13. Verfahren gemäß einem der Ansprüche 1 bis 12, **dadurch gekennzeichnet, dass** das Medium ein chemisch definiertes Medium ist.

14. Verfahren gemäß einem der Ansprüche 1 bis 13, **dadurch gekennzeichnet, dass** das Medium Protein-frei ist.

15. Verfahren gemäß einem der Ansprüche 1 bis 14, **dadurch gekennzeichnet, dass** die Vermehrung der Viren und der Zellen in einem Perfusionssystem durchgeführt wird.

16. Verfahren gemäß einem der Ansprüche 1 bis 15, **dadurch gekennzeichnet, dass** die Vermehrung der Viren und der Zellen in einem Batch-System durchgeführt wird.

17. Verfahren gemäß einem der Ansprüche 1 bis 16, **dadurch gekennzeichnet, dass** die Zellen für die Vermehrung der Viren und der Zellen bei einer Temperatur zwischen 30°-40°C gezogen werden.

18. Verfahren gemäß einem der Ansprüche 1 bis 17, **dadurch gekennzeichnet, dass** die Zellen für die Vermehrung der Viren bei einem Sauerstoff-Partialdruck zwischen 35%-60% gezogen werden.

19. Verfahren gemäß einem der Ansprüche 1 bis 18, **dadurch gekennzeichnet, dass** der pH-Wert des Mediums für die Vermehrung der Viren zwischen pH 6,8 und pH 7,8 liegt.

20. Verfahren gemäß einem der Ansprüche 1 bis 19, **dadurch gekennzeichnet, dass** das Virus in die Zellen durch Infektion mit einem m.o.i.-Wert zwischen 10⁻⁸ und 10 eingebracht wird.

21. Verfahren gemäß einem der Ansprüche 1 bis 20, **dadurch gekennzeichnet, dass** die Viren oder ein von diesen exprimiertes Protein aus dem Kulturüberstand oder den geernteten Zellen isoliert werden.

22. Verfahren gemäß Anspruch 21, **dadurch gekennzeichnet, dass** mindestens ein Teil des Kulturmediums für die Isolierung der Viren oder des Proteins von mindestens einem Teil der Zellen abgetrennt wird.

23. Verfahren gemäß Anspruch 22, **dadurch gekennzeichnet, dass** die Abtrennung mittels eines Tiefenfilters oder eines Seperators erfolgt.

24. Verfahren gemäß einem der Ansprüche 21 bis 23, **dadurch gekennzeichnet, dass** die. Reinigung eine Ultrazentrifugation zur Aufkonzentrierung umfasst.

25. Verfahren gemäß einem der Ansprüche 21 bis 24, **dadurch gekennzeichnet, dass** die Reinigung eine Chromatographie umfasst.

26. Verfahren gemäß einem der Ansprüche 21 bis 25, **dadurch gekennzeichnet, dass** die Viren während der Reinigung inaktiviert werden.

27. Verfahren gemäß einem der Ansprüche 1-26, **dadurch gekennzeichnet, dass** man die isolierten Viren oder das Protein mit einem geeigneten Adjuvanz, Hilfsstoff, Puffer, Verdünnungsmittel oder Arzneimittelträger vermischt.

## Claims

1. A method for preparing a pharmaceutical or diagnostic agent, comprising the steps of:
(a) infecting MDCK cells with a virus;
(b) culturing the MDCK cells after infection in cell culture under conditions that allow for a multiplication of the viruses and at the same time targeted additional, at least twofold multiplication of the cells by increasing the overall culture volume,
wherein the virus is selected from the group consisting of viruses of the European tick-borne encephalitis (TBE) and the related eastern (Russian or other) forms, Japanese encephalitis virus, dengue virus, yellow fever virus, rabies virus, hepatitis A virus or rotavirus.

2. A method according to claim 1, wherein the cells are cultured after infection under conditions that allow for an at least 5-fold, preferably at least a 10-fold multiplication of the cells.

3. A method according to claim 2, wherein the cells are cultured after infection in cell culture for at least 7, 21 or 28 days, preferably at least 35 days.

4. A method according to any of claims 1-3, wherein during the targeted multiplication of the viruses and cells fresh medium, medium concentrate or media components are added at least once, or at least a part of the viruses and cells are transferred to a culture vessel that contains fresh medium, medium concentrate or media components.

5. A method according to any of claims 1-4, wherein the addition of the fresh medium, medium concentrate or media components or the transfer of the cells and viruses into another culture vessel is repeated at least once.

6. A method according to any of claims 1-5, wherein the addition of the fresh medium, medium concentrate or media components or the transfer of the cells and viruses into another culture vessel is repeated several times.

7. A method according to any of claims 1-6, wherein medium is removed during multiplication of the viruses and the cells.

8. A method according to any of claims 1-7, wherein during multiplication of the viruses and the cells medium is continually removed and fresh medium, medium concentrate or media components are added.

9. A method according to any of claims 1-8, wherein the cells are cultured adherently or in suspension culture before infection.

10. A method according to any of claims 1-9, wherein the cells are cultured adherently or in suspension culture after infection.

11. A method according to any of claims 1-10, wherein the MDCK cells are derived from cell line MDCK 33016.

12. A method according to any of claims 1-11, wherein the medium is serum-free.

13. A method according to any of claims 1-12, wherein the medium is a chemically defined medium.

14. A method according to any of claims 1-13, wherein the medium is protein-free.

15. A method according to any of claims 1-14, wherein the multiplication of the viruses and the cells is carried out in a perfusion system.

16. A method according to any of claims 1-15, wherein the multiplication of the viruses and the cells is carried out in a batch system.

17. A method according to any of claims 1-16, wherein the cells are cultured at a temperature between 30°-40°C for the multiplication of the viruses and cells.

18. A method according to any of claims 1-17, wherein the cells are cultured at an oxygen partial pressure between 35%-60% for the multiplication of the viruses.

19. A method according to any of claims 1-18, wherein the pH of the medium is between 6.8 und 7.8 for the multiplication of the viruses.

20. A method according to any of claims 1-19, wherein the virus is introduced into the cells by infection at a m.o.i.-value between 10⁻⁸ and 10.

21. A method according to any of claims 1-20, wherein the viruses or a protein expressed by these viruses is isolated from the culture supernatant or from the harvested cells.

22. A method according to claim 21, wherein at least part of the culture medium is separated from at least part of the cells for the isolation of the viruses or the protein.

23. A method according to claim 22, wherein the separation is carried out by using a deep bed filter or a separator.

24. A method according to any of claims 21-23, wherein the purification comprises an ultracentrifugation for concentration.

25. A method according to any of claims 21-24, wherein the purification comprises a chromatography.

26. A method according to any of claims 21-25, wherein the viruses are inactivated during purification.

27. A method according to any of claims 1-26, wherein the isolated viruses or protein are mixed with an appropriate adjuvant, excipient, buffer, diluent or pharmaceutical carrier.

## Revendications

1. Procédé de préparation d'un médicament ou d'un agent de diagnostic, comprenant les étapes consistant à :
(a) infecter des cellules MDCK avec un virus ;
(b) après l'infection, soumettre les cellules MDCK à une culture cellulaire dans des conditions permettant la multiplication des virus ainsi que, en même temps, une multiplication ciblée des cellules, correspondant au moins à un doublement, par une augmentation du volume total de la culture,
dans lequel le virus est choisi dans le groupe constitué par les virus de la méningo-encéphalite à tique européenne ainsi que par les formes orientales apparentées (russes ou autres), le virus de l'encéphalite japonaise, le virus de la dengue, le virus de la fièvre jaune, les virus rabiques, les virus de l'hépatite A ou les rotavirus.

2. Procédé selon la revendication 1, **caractérisé en ce que** les cellules sont cultivées après l'infection dans des conditions qui permettent une multiplication au moins par 5, de préférence au moins par 10 des cellules.

3. Procédé selon la revendication 2, **caractérisé en ce que,** après l'infection, les cellules sont soumises à une culture cellulaire pendant au moins 7, 21 ou 28 jours, de préférence pendant au moins 35 jours.

4. Procédé selon l'une des revendications 1 à 3, **caractérisé en ce que,** pendant la multiplication ciblée des virus et des cellules, on ajoute au moins une fois du milieu, du concentré de milieu ou des constituants du milieu frais ou bien on transfère au moins une partie des virus et des cellules dans un récipient de culture qui contient du milieu, du concentré de milieu ou des constituants du milieu frais.

5. Procédé selon l'une des revendications 1 à 4, **caractérisé en ce que** l'ajout du milieu, du concentré de milieu ou des constituants du milieu ou le transfert des cellules et des virus dans un autre récipient de culture est répété au moins une fois.

6. Procédé selon l'une des revendications 1 à 5, **caractérisé en ce que** l'ajout du milieu, du concentré de milieu ou des constituants du milieu ou le transfert des cellules et des virus dans un autre récipient de culture est répété à plusieurs reprises.

7. Procédé selon l'une des revendications 1 à 6, **caractérisé en ce que** du milieu est prélevé pendant la multiplication des virus et des cellules.

8. Procédé selon l'une des revendications 1 à 7, **caractérisé en ce que** du milieu est prélevé et du milieu, du concentré de milieu ou des constituants du milieu frais ajouté en continu pendant la multiplication des virus et des cellules.

9. Procédé selon l'une des revendications 1 à 8, **caractérisé en ce que,** avant l'infection, les cellules sont cultivées jusqu'à l'adhérence ou sous la forme d'une culture en suspension.

10. Procédé selon l'une des revendications 1 à 9, **caractérisé en ce que,** après l'infection, les cellules sont cultivées jusqu'à l'adhérence ou sous la forme d'une culture en suspension.

11. Procédé selon l'une des revendications 1 à 10, **caractérisé en ce que** les cellules MDCK sont issues de la lignée cellulaire MDCK-33016.

12. Procédé selon l'une des revendications 1 à 11, **caractérisé en ce que** le milieu est dépourvu de sérum.

13. Procédé selon l'une des revendications 1 à 12, **caractérisé en ce que** le milieu est un milieu chimiquement défini.

14. Procédé selon l'une des revendications 1 à 13, **caractérisé en ce que** le milieu est dépourvu de protéines.

15. Procédé selon l'une des revendications 1 à 14, **caractérisé en ce que** la multiplication des virus et des cellules se fait dans un système de perfusion.

16. Procédé selon l'une des revendications 1 à 15, **caractérisé en ce que** la multiplication des virus et des cellules se fait dans un système par lots.

17. Procédé selon l'une des revendications 1 à 16, **caractérisé en ce que,** pour la multiplication des virus et des cellules, les cellules sont cultivées à une température dans la plage de 30 à 40°C.

18. Procédé selon l'une des revendications 1 à 17, **caractérisé en ce que,** pour la multiplication des virus, les cellules sont cultivées à une pression d'oxygène partielle dans la plage de 35% à 60%.

19. Procédé selon l'une des revendications 1 à 18, **caractérisé en ce que** le pH du milieu de multiplication des virus se situe dans la plage de pH 6,8 à pH 7,8.

20. Procédé selon l'une des revendications 1 à 19, **caractérisé en ce que** le virus est introduit dans les cellules par infection avec une valeur de multiplicité d'infection (MDI) dans la plage de 10⁻⁸ à 10.

21. Procédé selon l'une des revendications 1 à 20, **caractérisé en ce que** les virus ou une protéine exprimée par ceux-ci sont isolés à partir du surnageant de culture ou des cellules récoltées.

22. Procédé selon la revendication 21, **caractérisé en ce qu'**au moins une partie du milieu de culture est séparée d'au moins une partie des cellules pour isoler les virus ou la protéine.

23. Procédé selon la revendication 22, **caractérisé en ce que** la séparation s'effectue au moyen d'un filtre à lit profond ou d'un séparateur.

24. Procédé selon l'une des revendications 21 à 23, **caractérisé en ce que** la purification comprend une ultracentrifugation pour concentrer le produit.

25. Procédé selon l'une des revendications 21 à 24, **caractérisé en ce que** la purification comprend une chromatographie.

26. Procédé selon l'une des revendications 21 à 25, **caractérisé en ce que** les virus sont inactivés pendant la purification.

27. Procédé selon l'une des revendications 1 à 26, **caractérisé en ce que** l'on mélange les virus isolés ou la protéine avec un adjuvant, additif, tampon, diluant ou véhicule médicamenteux approprié.
